# EUROPÄISCHE PATENTSCHRIFT

(11) **EP 0 586 405 B1**
(45) Veröffentlichungstag und Bekanntmachung des Hinweises auf die Patenterteilung: **10.09.1997**
(21) Anmeldenummer: 92909456.3
(22) Anmeldetag: 06.05.1992
(51) Int. Cl.: C07D 405/04, C07D 311/22, C07D 213/30, C07D 213/89, C07D 493/04, C07D 239/26, C07F 9/58, A61K 31/44, A61K 31/47, A61K 31/505

(54) **NEUE IN 4-STELLUNG DURCH ARYL ODER N-HETEROARYL SUBSTITUIERTE 2H-BENZO[b]PYRAN-DERIVATE, VERFAHREN ZU IHRER HERSTELLUNG UND IHRE VERWENDUNG SOWIE DIE VERBINDUNGEN ENTHALTENDE ZUBEREITUNGEN**
NOVEL 2H-BENZO[b]PYRANE DERIVATIVES SUBSTITUTED IN THE 4 POSITION BY ARYL OR N-HETEROARYL, PROCESS FOR PRODUCING THEM AND PREPARATIONS CONTAINING THE COMPOUNDS
NOUVEAUX DERIVES DU 2H-BENZO[b]PYRANNE SUBSTITUES EN POSITION 4 PAR UN ARYLE OU UN N-HETEROARYLE, LEUR PROCEDE DE FABRICATION ET LEUR APPLICATION ET PREPARATIONS CONTENANT CES COMPOSES

(30) Priorität: 11.05.1991 DE 4115465
(43) Veröffentlichungstag der Anmeldung: 16.03.1994
(73) Patentinhaber: Beiersdorf-Lilly GmbH, 20253 Hamburg (DE)
(72) Erfinder: ARMAH, Ben, D-2000 Hamburg 52 (DE); MUSTER, Dieter, D-1000 Berlin 41 (DE); RÜHTER, Gerd, D-2101 Hamburg 96 (DE); SCHOTTEN, Theo, D-2000 Hamburg 20 (DE); STENZEL, Wolfgang, D-2057 Reinbek (DE)
(74) Vertreter: Hudson, Christopher Mark
(86) Internationale Anmeldenummer: DE9200356
(87) Internationale Veröffentlichungsnummer: WO9220671

(56) Entgegenhaltungen:
- EP-A- 0 296 975
- EP-A- 0 298 452
- EP-A- 0 350 805

## Beschreibung

Gegenstand der Erfindung sind neue substituierte Benzopyran-Derivate der allgemeinen Formel I, 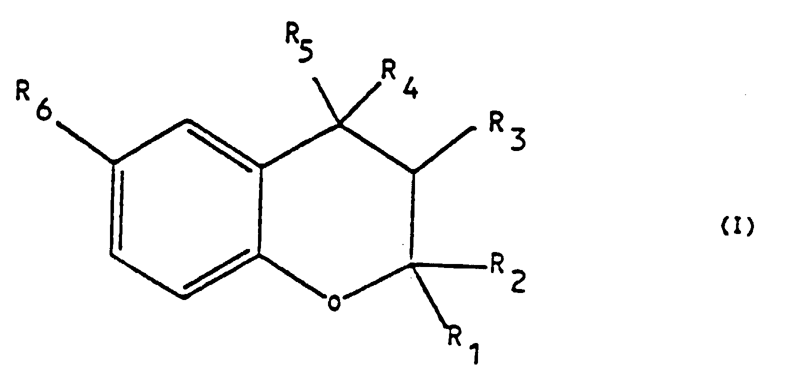 in der
R₁ und R₂, die gleich oder verschieden sein können, Wasserstoff, C₁₋₆-Alkyl, C₃₋₆-verzweigt-Alkyl, C₃₋₇-Cycloalkyl, Phenyl oder zusammen mit dem von ihnen eingeschlossenen Kohlenstoffatom C₃₋₇-Spiroalkyl bedeuten,
entweder R₃ oder R₄ Wasserstoff, Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und der jeweils andere Substituent der beiden Wasserstoff ist, oder R₃ und R₄ zusammen eine Bindung bilden,
R₅ eine monocyclische sechsgliedrige oder eine bicyclische aus zwei kondensierten Sechsringen bestehende Aryl- oder N-Heteroarylgruppe, in der der Heterocyclus ein oder zwei Stickstoffatome enthält, bedeutet, die einer der folgenden zwei Untergruppen A) oder B) angehört, wobei in
   A) R₅ eine N-Heteroarylgruppe mit einem oder zwei N-Atomen ist, die in der 2-Stellung eine N-Oxidgruppe trägt und gegebenenfalls ein- oder zweifach durch Halogen, C₁₋₈-Alkyl, C₁₋₈-Alkoxy, C₁₋₈-Mono- oder C₁₋₈-Dialkylamino, Hydroxy, Amino, Cyan, C₁₋₈-Alkoxycarbonyl, C₁₋₈-Mono- oder C₁₋₈-Dialkylaminocarbonyl, Hydroxycarbonyl, Aminocarbonyl, Phenyl substituiert ist, und in
   B) R₅ eine N-Heteroarylgruppe oder ein N-Heteroaryl-N-oxid ist, deren Grundkörper aus der Gruppe 3-Pyridinyl, 4-Pyridinyl, 5-Pyrimidinyl, 3-Pyridazinyl, 4-Pyrimidinyl, 2-Pyrazinyl, 3-Chinolinyl, 4-Isochinolinyl stammt und gegebenenfalls ein- oder zweifach durch Halogen, C₁₋₈-Alkyl, C₁₋₈-Alkoxy, C₁₋₈-Monoalkylamino, C₁₋₈-Dialkylamino, Cyan, Hydroxy, Amino, Phenyl substituiert ist, wobei die Hydroxy- oder die N-Oxidgruppe in dieser Untergruppe sich nicht in der 2-Stellung befindet, und
R₆ Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl, Trifluorethylsulfonyl, Phosphono oder C₁₋₈-Dialkoxyphosphoryl ist, wobei vorstehend genanntes Alkyl geradkettig oder verzweigt sein kann, sowie deren Salze und Säureadditionssalze, Tautomere und optische Isomere, Verfahren zu ihrer Herstellung, ihre Verwendung und Zubereitungen, die diese Verbindungen enthalten.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Verbindungen der allgemeinen Formel I und deren Salze und Säureadditionssalze enthalten asymmetrische Kohlenstoff- und Schwefelatome. Es sind daher auch die verschiedenen optischen Isomeren sowie Diastereomeren Gegenstand der Erfindung. Die Racemate können nach an sich bekannten Methoden in ihre optischen Antipoden aufgetrennt werden.

Gegenstand der Erfindung sind auch die neuen Verbindungen der Formeln II, IV, V und XI: 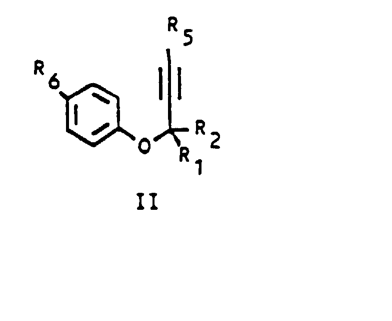 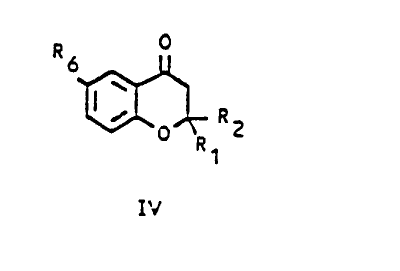 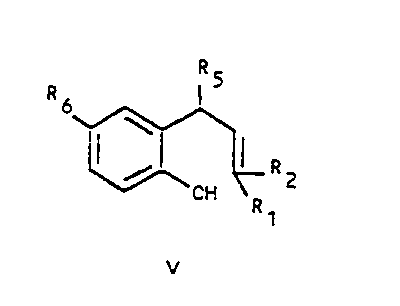 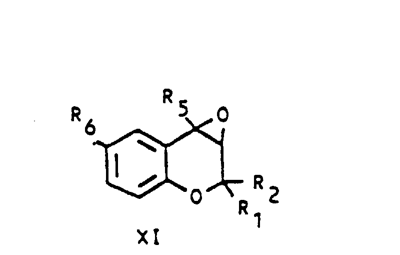 mit den vorstehend angegebenen Bedeutungen für R₁, R₂, R₅ und R₆ und die Verfahren zu ihrer Herstellung. Sie dienen als Vorprodukte oder Zwischenprodukte zur Herstellung der erfindungsgemäßen Endprodukte.

Mit den Verbindungen der vorliegenden Erfindung strukturell verwandte Verbindungen sind in der europäischen Patentanmeldung 298 452 beschrieben. Die Verbindungen der vorliegenden Erfindung werden aber weder spezifisch offenbart noch nahegelegt.

Soweit nicht anders angegeben, können die erfindungsgemäßen Alkylgruppen und Alkylteile beziehungsweise Alkylenteile von Gruppen geradkettig oder verzweigt sein, und sie besitzen jeweils bevorzugt 1 bis 6 Kohlenstoffatome, vorzugsweise 1 bis 4 Kohlenstoffatome, insbesondere 1 oder 2 Kohlenstoffatome. Die verzweigten Alkylgruppen besitzen mindestens 3 Kohlenstoffatome. Bevorzugte Alkyl- oder Alkylenteile sind Methyl, Ethyl, n-Propyl, Isopropyl, Butyl beziehungsweise entsprechend Methylen, Ethylen, n- oder Isopropylen und Butylen.

Vorzugsweise besitzen erfindungsgemäße Cycloalkylgruppen 3 bis 7 Kohlenstoffatome, insbesondere 3 bis 6 Kohlenstoffatome. Besonders bevorzugt sind Cyclopropyl, Cyclopentyl und Cyclohexyl.

Formyl ist HCO-, Formyloxy ist HCOO-, C₁₋₈-Alkylcarbonyloxy ist C₁₋₈-Alkyl-CO-O-, C₁₋₈-Alkoxycarbonyloxy ist C₁₋₈-Alkyl-O-CO-O-, C₁₋₈-Monoalkylaminocarbonyloxy ist C₁₋₈-Alkyl-NH-CO-O-, C₁₋₈-Dialkylaminocarbonyloxy ist (C₁₋₈-Alkyl)₂N-CO-O-, C₁₋₈-Alkylcarbonyl ist C₁₋₈-Alkyl-CO-, C₁₋₈-Alkoxycarbonyl ist C₁₋₈-Alkoxy-CO-, C₁₋₈-Monoalkylaminocarbonyl ist C₁₋₈-Alkyl-NH-CO-, C₁₋₈-Dialkylaminocarbonyl ist (C₁₋₈-Alkyl)₂N-CO-, C₁₋₈-Dialkylamino ist (C₁₋₈-Alkyl)₂N-, C₁₋₈-Monoalkylamino ist C₁₋₈-Alkyl-NH-, Halogen ist Fluor, Chlor, Brom, Jod, Phosphono bedeutet -PO(OH)₂ und C₁₋₈-Dialkoxyphosphoryl ist (C₁₋₈-Alkyl-O)₂PO-.

Die Trifluorethylgruppe oder Trifluorethyl als Teil anderer erfindungsgemäßer Reste wie Trifluorethoxy ist vorzugsweise 2,2,2-Trifluorethyl.

Die Tetrafluorethylgruppe oder Tetrafluorethyl als Teil anderer erfindungsgemäßer Reste wie Tetrafluorethoxy ist vorzugsweise 1,1,2,2-Tetrafluorethyl.

Halogen ist vorzugsweise Fluor oder Chlor.

R₁ und R₂ sind bevorzugt Wasserstoff, Methyl oder Ethyl, davon besonders bevorzugt Methyl.

R₁ und R₂ sind ganz besonders bevorzugt beide zugleich Methyl.

Falls R₁ und R₂ zusammen mit dem von ihnen eingeschlossenen Kohlenstoffatom einen Spiroalkylring bilden, sind Spirocyclopentyl und Spirocyclohexyl bevorzugt.

R₃ steht bevorzugt für Wasserstoff oder Hydroxy, wenn R₄ Wasserstoff ist, und R₄ steht bevorzugt für Wasserstoff oder Hydroxy, wenn R₃ Wasserstoff ist. Bevorzugt bilden auch R₃ und R₄ eine Bindung, so daß zwischen dem C-3 und dem C-4 des Benzopyrangerüstes eine Doppelbindung besteht.

Wenn R₃ nicht Wasserstoff ist, sondern die oben angegebene Bedeutung hat, und nicht zusammen mit R₄ eine Bindung bildet, so stehen die Substituenten R₃ und R₅ in Verbindungen der allgemeinen Formel I bevorzugt trans zueinander.

Falls R₃ oder R₄ für Alkoxy steht, sind Ethoxy und besonders Methoxy bevorzugt.

Falls R₃ oder R₄ für Alkylcarbonyloxy steht, sind Propionyloxy und besonders Acetoxy und Formyloxy bevorzugt.

Ganz besonders bevorzugt bilden R₃ und R₄ zwischen dem C-3 und dem C-4 des Benzopyrangerüstes eine Bindung.

Monocyclische oder bicyclische Aryl- oder N-Heteroarylgruppen, die den Untergruppen A) und B) zugrunde liegen, sind insbesondere Phenyl, Naphthyl, Pyridinyl, Pyrimidinyl, Pyridazinyl, Pyrazinyl, Chinolinyl, Isochinolinyl, besonders bevorzugt aber Pyridinyl und Pyrimidinyl.

Bevorzugte mono- oder bicyclische N-Heteroarylgruppen R₅ der Untergruppe A) sind 2-Pyridinyl-N-oxid, 3-Chlor-2-pyridinyl-N-oxid, 4-Chlor-2-pyridinyl-N-oxid, 5-Chlor-2-pyridinyl-N-oxid, 6-Chlor-2-pyridinyl-N-oxid, 3-Methyl-2-pyridinyl-N-oxid, 4-Methyl-2-pyridinyl-N-oxid, 5-Methyl-2-pyridinyl-N-oxid, 6-Methyl-2-pyridinyl-N-oxid, 5-Phenyl-2-pyridinyl-N-oxid, 4-Hydroxy-2-pyridinyl-N-oxid, 5-Hydroxy-2-pyridinyl-N-oxid, 6-Hydroxy-2-pyridinyl-N-oxid, 4-Methoxy-2-pyridinyl-N-oxid, 5-Methoxy-2-pyridinyl-N-oxid, 6-Methoxy-2-pyridinyl-N-oxid, 5-Methoxycarbonyl-2-pyridinyl-N-oxid, 5-Cyano-2-pyridinyl-N-oxid, 2-Pyrimidinyl-1-oxid, 6-Pyrimidinyl-1-oxid, 2-Pyrazinyl-1-oxid, 2-Chinolinyl-N-oxid, 3-Isochinolinyl-N-oxid.

Bevorzugte mono- oder bicyclische N-Heteroarylgruppen R₅ der Untergruppe B) sind 3-Pyridinyl, 3-Pyridinyl N-oxid, 4-Pyridinyl, 4-Pyridinyl-N-oxid, 2-Methyl-3-pyridinyl, 4-Methyl-3-pyridinyl, 5-Methyl-3-pyridinyl, 6-Methyl-3-pyridinyl, 2-Methoxy-3-pyridinyl, 4-Methoxy-3-pyridinyl, 5-Methoxy-3-pyridinyl, 6-Methoxy-3-pyridinyl, 2-Fluor-3-pyridinyl, 2-Chlor-3-pyridinyl, 6-Chlor-3-pyridinyl, 2-Cyano-3-pyridinyl, 6-Cyano-3-pyridinyl, 6-Methoxycarbonyl-3-pyridinyl, 6-Dimethylamino-3-pyridinyl, 6-Hydroxy-3-pyridinyl, 3-Pyridazinyl, 6-Methyl-3-pyridazinyl, 2-Pyrazinyl, 2-Pyrazinyl-4-oxid, 5-Methyl-2-pyrazinyl, 6-Methyl-2-pyrazinyl, 5,6-Dimethyl-2-pyrazinyl, 4-Pyrimidinyl, 5-Pyrimidinyl, 5-Pyrimidinyl-1-oxid, 3-Chinolinyl, 3-Chinolinyl-N-oxid, 4-Isochinolinyl, 4-Isochinolinyl-N-oxid.

Erfindungsgemäße Verbindungen mit Substituenten der Untergruppen A) und B) werden bevorzugt, besonders bevorzugt aber solche mit den folgenden Substituenten:
2-Pyridinyl-N-oxid, 2-Pyrimidinyl-N-oxid, 2-Pyrazinyl-1-oxid, 6-Pyrimidinyl-1-oxid, 2-Chinolinyl-N-oxid, 3-Isochinolinyl-N-oxid, 3-Pyridinyl, 3-Pyridinyl-N-oxid, 2-Fluor-3-pyridinyl, 5-Pyrimidinyl, 3-Pyridazinyl, 3-Chinolinyl, 4-Isochinolyl.

Ganz besonders bevorzugte Substituenten R₅ sind 2-Pyridinyl-N-oxid und 3-Pyridinyl.

Ist R₆ C₁₋₈-Dialkoxyphosphoryl, so können die Alkoxyreste gleich oder verschieden sein. Bevorzugt sind sie beide zugleich Methoxy oder Ethoxy.

Bevorzugte Substituenten R₆ sind Difluormethoxy, Trifluormethoxy, Trifluormethylthio, Trifluormethylsulfonyl und Diethoxyphosphoryl.

Bevorzugte Verbindungen der allgemeinen Formel I sind Verbindungen der allgemeinen Formel VI. Besonders bevorzugte Verbindungen der allgemeinen Formel VI sind solche, in denen R₁ und R₂ beide zugleich Methyl sind und R₅ für 2-Pyridinyl-N-oxid oder 3-Pyridinyl steht.

Die folgenden erfindungsgemäßen Verbindungen, deren Salze und Säureadditionssalze, Tautomere und optische Isomere werden bevorzugt:
1. 6-Difluormethoxy-2,2-dimethyl-4-(2'-pyridinyl)-2H-benzo[b]pyran-1'-oxid
2. 6-Trifluormethoxy-2,2-dimethyl-4-(2'-pyridinyl)-2H-benzo[b]pyran-1'-oxid
3. 6-Trifluormethylthio-2,2-dimethyl-6-(2'-pyridinyl)-2H-benzo[b]pyran-1'-oxid
4. 6-Trifluormethylsulfonyl-2,2-dimethyl-4-(2'-pyridinyl)-2H-benzo[b]pyran-1'-oxid
5. 6-Trifluormethylsulfinyl-2,2-dimethyl-4-(2'-pyridinyl)-2H-benzo[b]pyran-1'-oxid
6. 6-Difluormethylthio-2,2-dimethyl-4-(2'-pyridinyl)-2H-benzo[b]pyran-1'-oxid
7. 6-Difluormethylsulfonyl-2,2-dimethyl-4-(2'-pyridinyl)-2H-benzo[b]pyran-1'-oxid
8. 6-Difluormethylsulfinyl-2,2-dimethyl-4-(2'-pyridinyl)-2H-benzo[b]pyran-1'-oxid
9. 6-(2',2',2'-Trifluorethoxy)-2,2-dimethyl-4-(2'-pyridinyl) 2H-benzo[b]pyran-1'-oxid
10. 6-(2',2',2'-Trifluorethylthio)-2,2-dimethyl-4-(2'-pyridinyl)-2H-benzo[b]pyran-1'-oxid
11. 6-(2',2',2'-Trifluorethylsulfonyl)-2,2-dimethyl-4-(2'-pyridinyl)-2H-benzo[b]pyran-1'-oxid
12. 6-(2',2',2'-Trifluorethylsulfinyl)-2,2-dimethyl-4-(2'-pyridinyl)-2H-benzo[b]pyran-1'-oxid
13. 6-(1',1',2',2'-Tetrafluorethoxy)-2,2-dimethyl-4-(2'-pyridinyl)-2H-benzo[b]pyran-1'-oxid
14. 6-Diethoxyphosphoryl-2,2-dimethyl-4-(2'-pyridinyl)-2H-benzo[b]pyran-1'-oxid
15. 6-Difluormethoxy-2,2-dimethyl-4-(2'-pyridinyl)-3,4-dihydro-2H-benzo[b]pyran-1'-oxid
16. 6-Trifluormethoxy-2,2-dimethyl-4-(2'-pyridinyl)-3,4-dihydro-2H-benzo[b]pyran-1'-oxid
17. 6-Trifluormethylthio-2,2-dimethyl-4-(2'-pyridinyl)-3,4-dihydro-2H-benzo[b]pyran-1'-oxid
18. 6-Trifluormethylsulfonyl-2,2-dimethyl-4-(2'-pyridinyl)-3,4-dihydro-2H-benzo[b]pyran-1'-oxid
19. 6-Trifluormethylsulfinyl-2,2-dimethyl-4-(2'-pyridinyl)-3,4-dihydro-2H-benzo[b]pyran-1'-oxid
20. 6-Difluormethylthio-2,2-dimethyl-4-(2'-pyridinyl)-3,4-dihydro-2H-benzo[b]pyran-1'-oxid
21. 6-Difluormethylsulfonyl-2,2-dimethyl-4-(2'-pyridinyl)-3,4-dihydro-2H-benzo[b]pyran-1'-oxid
22. 6-Diethoxyphosphoryl-2,2-dimethyl-4-(2'-pyridinyl)-3,4-dihydro-2H-benzo[b]pyran-1'-oxid
23. 6-Trifluormethoxy-2,2-dimethyl-4-(2'-pyridinyl)-3,4-dihydro-2H-benzo[b]pyran-4-ol-1'-oxid
24. 6-Difluormethoxy-2,2-dimethyl-4-(2'-pyridinyl)-3,4-dihydro-2H-benzo[b]pyran-4-ol-1'-oxid
25. 6-Trifluormethylthio-2,2-dimethyl-4-(2'-pyridinyl)-3,4-dihydro-2H-benzo[b]pyran-4-ol-1'-oxid
26. 6-Trifluormethylsulfonyl-2,2-dimethyl-4-(2'-pyridinyl)-3,4-dihydro-2H-benzo[b]pyran-4-ol-1'-oxid
27. 6-Trifluormethylsulfinyl-2,2-dimethyl-4-(2'-pyridinyl)-3,4-dihydro-2H-benzo[b]pyran-4-ol-1'-oxid
28. 6-Trifluormethylthio-2,2-dimethyl-4-methoxy-4-(2'-pyridinyl)-3,4-dihydro-2H-benzo[b]pyran-1'-oxid
29. 6-Trifluormethylsulfonyl-2,2-dimethyl-4-methoxy-4-(2'-pyridinyl)-3,4-dihydro-2H-benzo[b]pyran-1'-oxid
30. 6-Trifluormethylsulfinyl-2,2-dimethyl-4-methoxy-4-(2'-pyridinyl)-3,4-dihydro-2H-benzo[b]pyran-1'-oxid
31. 6-Difluormethoxy-2,2-dimethyl-4-(2'-pyridinyl)trans-3,4-dihydro-2H-benzo[b]pyran-3-ol-1'-oxid
32. 6-Trifluormethoxy-2,2-dimethyl-4-(2'-pyridinyl)trans-3,4-dihydro-2H-benzo[b]pyran-3-ol-1'-oxid
33. 6-Trifluormethylthio-2,2-dimethyl-4-(2'-pyridinyl)trans-3,4-dihydro-2H-benzo[b]pyran-3-ol-1'-oxid
34. 6-Trifluormethylsulfonyl-2,2-dimethyl-4-(2'-pyridinyl)trans-3,4-dihydro-2H-benzo[b]pyran-3-ol-1'-oxid
35. 6-Trifluormethylthio-2,2-dimethyl-4-(3'-chlor-2'-pyridinyl)-2H-benzo[b]pyran-1'-oxid
36. 6-Trifluormethylsulfonyl-2,2-dimethyl-4-(3'-chlor-2'-pyridinyl)-2H-benzo[b]pyran-1'-oxid
37. 6-Difluormethoxy-2,2-dimethyl-4-(4'-chlor-2'-pyridinyl)-2H-benzo[b]pyran-1'-oxid
38. 6-Trifluormethylthio-2,2-dimethyl-4-(4'-chlor-2'-pyridinyl)-2H-benzo[b]pyran-1'-oxid
39. 6-Trifluormethylsulfonyl-2,2-dimethyl-4-(4'-chlor-2'-pyridinyl)-2H-benzo[b]pyran-1'-oxid
40. 6-Difluormethoxy-2,2-dimethyl-4-(5'-chlor-2'-pyridinyl)-2H-benzo[b]pyran-1'-oxid
41. 6-Trifluormethylthio-2,2-dimethyl-4-(5'-chlor-2'-pyridinyl)-2H-benzo[b]pyran-1'-oxid
42. 6-Trifluormethylsulfonyl-2,2-dimethyl-4-(5'-chlor-2'-pyridinyl)-2H-benzo[b]pyran-1'-oxid
43. 6-Trifluormethylsulfonyl-2,2-dimethyl-4-(6'-chlor-2'-pyridinyl)-2H-benzo[b]pyran-1'-oxid
44. 6-Trifluormethylthio-2,2-dimethyl-4-(3'-methyl-2'-pyridinyl)-2H-benzo[b]pyran-1'-oxid
45. 6-Difluormethoxy-2,2-dimethyl-4-(4'-methyl-2'-pyridinyl)-2H-benzo[b]pyran-1'-oxid
46. 6-Trifluormethylsulfonyl-2,2-dimethyl-4-(4'-methyl-2'-pyridinyl)-2H-benzo[b]pyran-1'-oxid
47. 6-Difluormethoxy-2,2-dimethyl-4-(5'-methyl-2'-pyridinyl)-2H-benzo[b]pyran-1'-oxid
48. 6-Trifluormethylthio-2,2-dimethyl-4-(5'-methyl-2'-pyridinyl)-2H-benzo[b]pyran-1'-oxid
49. 6-Trifluormethylsulfonyl-2,2-dimethyl-4-(5'-methyl-2'-pyridinyl)-2H-benzo[b]pyran-1'-oxid
50. 6-Trifluormethoxy-2,2-dimethyl-4-(5'-methyl-2'-pyridinyl)-2H-benzo[b]pyran-1'-oxid
51. 6-Trifluormethylsulfonyl-2,2-dimethyl-4-(6'-methyl-2'-pyridinyl)-2H-benzo[b]pyran-1'-oxid
52. 6-Trifluormethylthio-2,2-dimethyl-4-(5'-phenyl-2'-pyridinyl)-2H-benzo[b]pyran-1'-oxid
53. 6-Trifluormethylsulfonyl-2,2-dimethyl-4-(5'-phenyl-2'-pyridinyl)-2H-benzo[b]pyran-1'-oxid
54. 6-Trifluormethylthio-2,2-dimethyl-4-(4'-hydroxy-2'-pyridinyl)-2H-benzo[b]pyran-1'-oxid
55. 6-Trifluormethylthio-2,2-dimethyl-4-(5'-hydroxy-2'-pyridinyl)-2H-benzo[b]pyran-1'-oxid
56. 6-Trifluormethylthio-2,2-dimethyl-4-(6'-hydroxy-2'-pyridinyl)-2H-benzo[b]pyran-1'-oxid
57. 6-Trifluormethylthio-2,2-dimethyl-4-(4'-methoxy-2'-pyridinyl)-2H-benzo[b]pyran-1'-oxid
58. 6-Trifluormethylthio-2,2-dimethyl-4-(5'-methoxy-2'-pyridinyl)-2H-benzo[b]pyran-1'-oxid
59. 6-Trifluormethylthio-2,2-dimethyl-4-(6'-methoxy-2'-pyridinyl)-2H-benzo[b]pyran-1'-oxid
60. 6-Difluormethoxy-2,2-dimethyl-4-(5'-methoxycarbonyl-2'-pyridinyl)-2H-benzo[b]pyran-1'-oxid
61. 6-Difluormethoxy-2,2-dimethyl-4-(5'-cyano-2'-pyridinyl)-2H-benzo[b]pyran-1'-oxid
62. 6-Difluormethoxy-2,2-dimethyl-4-(2'-pyrimidinyl)-2H-benzo[b]pyran-1'-oxid
63. 6-Difluormethoxy-2,2-dimethyl-4-(2'-pyrimidinyl)-3,4-dihydro-2H-benzo[b]pyran-1'-oxid
64. 6-Trifluormethoxy-2,2-dimethyl-4-(2'-pyrimidinyl)-2H-benzo[b]pyran-1'-oxid
65. 6-Trifluormethoxy-2,2-dimethyl-4-(2'-pyrimidinyl)-3,4-dihydro-2H-benzo[b]pyran-1'-oxid
66. 6-Trifluormethylsulfonyl-2,2-dimethyl-4-(2'-pyrimidinyl)-2H-benzo[b]pyran-1'-oxid
67. 6-Trifluormethylsulfonyl-2,2-dimethyl-4-(2'-pyrimidinyl)3,4-dihydro-2H-benzo[b]pyran-1'-oxid
68. 6-Difluormethoxy-2,2-dimethyl-4-(6'-pyrimidinyl)3,4-dihydro-2H-benzo[b]pyran-1'-oxid
69. 6-Trifluormethoxy-2,2-dimethyl-4-(6'-pyrimidinyl)-3,4-dihydro-2H-benzo[b]pyran-1'-oxid
70. 6-Trifluormethylsulfonyl-2,2-dimethyl-4-(6'-pyrimidinyl)-3,4-dihydro-2H-benzo[b]pyran-1'-oxid
71. 6-Difluormethoxy-2,2-dimethyl-4-(2'-pyrazinyl)-3,4-dihydro-2H-benzo[b]pyran-1'-oxid
72. 6-Trifluormethoxy-2,2-dimethyl-4-(2'-pyrazinyl)-3,4-dihydro-2H-benzo[b]pyran-1'-oxid
73. 6-Trifluormethylsulfonyl-2,2-dimethyl-4-(2'-pyrazinyl)-3,4-dihydro-2H-benzo[b]pyran-1'-oxid
74. 6-Difluormethoxy-2,2-dimethyl-4-(2'-chinolinyl)-2H-benzo[b]pyran-1'-oxid
75. 6-Trifluormethoxy-2,2-dimethyl-4-(2'-chinolinyl)-2H-benzo[b]pyran-1'-oxid
76. 6-Trifluormethylthio-2,2-dimethyl-4-(2'-chinolinyl)-2H-benzo[b]pyran-1'-oxid
77. 6-Trifluormethylsulfonyl-2,2-dimethyl-4-(2'-chinolinyl)-2H-benzo[b]pyran-1'-oxid
78. 6-Trifluormethylsulfinyl-2,2-dimethyl-4-(2'-chinolinyl)-2H-benzo[b]pyran-1'-oxid
79. 6-Trifluormethoxy-2,2-dimethyl-4-(3'-isochinolinyl)-2H-benzo[b]pyran-1'-oxid
80. 6-Trifluormethylthio-2,2-dimethyl-4-(3'-isochinolinyl)-2H-benzo[b]pyran-1'-oxid
81. 6-Trifluormethylsulfonyl-2,2-dimethyl-4-(3'-isochinolinyl) -2H-benzo[b]pyran-1'-oxid
88. 6-Trifluormethylthio-2,2-dimethyl-4-(1'-hydroxy-2'-naphthyl)-2H-benzo[b]pyran
89. 6-Trifluormethylthio-2,2-dimethyl-4-(3'-hydroxy-2'-naphthyl)-2H-benzo[b]pyran
92. 6-Trifluormethylthio-2,2-dimethyl-4-(4'-hydroxy-3'-pyridinyl)-2H-benzo[b]pyran
93. 6-Trifluormethylthio-2,2-dimethyl-1-(3'-hydroxy-4'-pyridinyl)-2H-benzo[b]pyran
94. 6-Trifluormethylthio-2,2-dimethyl-4-(3'-hydroxy-4'-pyridazinyl)-2H-benzo[b]pyran
95. 6-Trifluormethylthio-2,2-dimethyl-4-(3'-hydroxy-2'-pyrazinyl)-2H-benzo[b]pyran
97. 6-Difluormethoxy-2,2-dimethyl-4-(3'-pyridinyl)-2H-benzo[b]pyran
98. 6-Difluormethoxy-2,2-dimethyl-4-(3'-pyridinyl)-2H-benzo[b]pyran-1'-oxid
99. 6-Difluormethoxy-2,2-dimethyl-4-(3'-pyridinyl)-3,4-dihydro-2H-benzo[b]pyran
100. 6-Trifluormethoxy-2,2-dimethyl-4-(3'-pyridinyl)-2H-benzo[b]pyran
101. 6-Trifluormethoxy-2,2-dimethyl-4-(3'-pyridinyl)-2H-benzo[b]pyran-1'-oxid
102. 6-Trifluormethoxy-2,2-dimethyl-4-(3'-pyridinyl)-3,4-dihydro-2H-benzo[b]pyran
103. 6-Trifluormethylthio-2,2-dimethyl-4-(3'-pyridinyl)-2H-benzo[b]pyran
104. 6-Trifluormethylthio-2,2-dimethyl-4-(3'-pyridinyl)-2H-benzo[b]pyran-1'-oxid
105. 6-Trifluormethylthio-2,2-dimethyl-6-(3'-pyridinyl)-3,4-dihydro-2H-benzo[b]pyran
106. 6-Trifluormethylthio-2,2-dimethyl-4-(3'-pyridinyl)-3,4-dihydro-2H-benzo[b]pyran-4-ol
107. 6-Trifluormethylsulfonyl-2,2-dimethyl-4-(3'-pyridinyl)-2H-benzo[b]pyran
108. 6-Trifluormethylsulfonyl-2,2-dimethyl-4-(3'-pyridinyl)-2H-benzo[b]pyran-1'-oxid
109. 6-Trifluormethylsulfonyl-2,2-dimethyl-4-(3'-pyridinyl)-3,4-dihydro-2H-benzo[b]pyran
110. 6-Trifluormethylsulfonyl-2,2-dimethyl-4-(3'-pyridinyl)-3,4-dihydro-2H-benzo[b]pyran-4-ol
111. 6-Diethoxyphosphoryl-2,2-dimethyl-4-(3'-pyridinyl)-2H-benzo[b]pyran
112. 6-Diethoxyphoshoryl-2,2-dimethyl-4-(3'-pyridinyl)-2H-benzo[b]pyran-1'-oxid
113. 6-Diethoxyphosphoryl-2,2-dimethyl-4-(3'-pyridinyl)-3,4-dihydro-2H-benzo[b]pyran
114. 6-Difluormethoxy-2,2-dimethyl-4-(4'-pyridinyl)-2H-benzo[b]pyran
115. 6-Trifluormethoxy-2,2-dimethyl-4-(4'-pyridinyl)-2H-benzo[b]pyran
116. 6-Trifluormethoxy-2,2-dimethyl-4-(4'-pyridinyl)-2H-benzo[b]pyran-1'-oxid
117. 6-Trifluormethylthio-2,2-dimethyl-4-(4'-pyridinyl)-2H-benzo[b]pyran
118. 6-Trifluormethylsulfonyl-2,2-dimethyl-4-(4'-pyridinyl)-2H-benzo[b]pyran
119. 6-Trifluormethylsulfonyl-2,2-dimethyl-4-(4'-pyridinyl)-2H-benzo[b]pyran-1'-oxid
120. 6-Trifluormethylsulfonyl-2,2-dimethyl-4-(2'-methyl-3'-pyridinyl)-2H-benzo[b]pyran
121. 6-Trifluormethylsulfonyl-2,2-dimethyl-4-(4'-methyl-3'-pyridinyl)-2H-benzo[b]pyran
122. 6-Trifluormethylsulfonyl-2,2-dimethyl-4-(5'-methyl-3'-pyridinyl)-2H-benzo[b]pyran
123. 6-Trifluormethylsulfonyl-2,2-dimethyl-4-(6'-methyl-3'-pyridinyl)-2H-benzo[b]pyran
124. 6-Trifluormethylthio-2,2-dimethyl-4-(2'-methoxy-3'-pyridinyl)-2H-benzo[b]pyran
125. 6-Trifluormethylthio-2,2-dimethyl-4-(2'-methoxy-3'-pyridinyl)-2H-benzo[b]pyran-4-ol
126. 6-Trifluormethylthio-2,2-dimethyl-4-(4'-methoxy-3'-pyridinyl)-2H-benzo[b]pyran
127.- 6-Trifluormethylthio-2,2-dimethyl-4-(4'-methoxy-3'-pyridinyl)-2H-benzo[b]pyran-4-ol
128. 6-Trifluormethylthio-2,2-dimethyl-4-(5'-methoxy-3'-pyridinyl)-2H-benzo[b]pyran
129. 6-Trifluormethylthio-2,2-dimethyl-4-(5'-methoxy-3'-pyridinyl)-2H-benzo[b]pyran-4-ol
130. 6-Trifluormethylthio-2,2-dimethyl-4-(6'-methoxy-3'-pyridinyl)-2H-benzo[b]pyran
131. 6-Trifluormethylthio-2,2-dimethyl-4-(6'-methoxy-3'-pyridinyl)-2H-benzo[b]pyran-4-ol
132. 6-Trifluormethylthio-2,2-dimethyl-4-(2'-fluor-3'-pyridinyl)-2H-benzo[b]pyran
133. 6-Trifluormethylthio-2,2-dimethyl-4-(2'-fluor-3'-pyridinyl)-2H-benzo[b]pyran-4-ol
134. 6-Trifluormethoxy-2,2-dimethyl-4-(2'-chlor-3'-pyridinyl)-2H-benzo[b]pyran
135. 6-Trifluormethoxy-2,2-dimethyl-4-(6'-chlor-3'-pyridinyl)-2H-benzo[b]pyran
136. 6-Trifluormethoxy-2,2-dimethyl-4-(2'-cyano-3'-pyridinyl)-2H-benzo[b]pyran
137. 6-Trifluormethoxy-2,2-dimethyl-4-(6'-cyano-3'-pyridinyl)-2H-benzo[b]pyran
138. 6-Trifluormethoxy-2,2-dimethyl-4-(6'-methoxy-carbonyl-3'-pyridinyl)-2H-benzo[b]pyran
139. 6-Trifluormethoxy-2,2-dimethyl-4-(6'-dimethylamino-3'-pyridinyl)-2H-benzo[b]pyran
140. 6-Trifluormethoxy-2,2-dimethyl-4-(6'-hydroxy-3'-pyridinyl)-2H-benzo[b]pyran
141. 6-Trifluormethylthio-2,2-dimethyl-4-(3'-pyridezinyl)-2H-benzo[b]pyran
142. 6-Trifluormethylthio-2,2-dimethyl-4-(6'-methyl-3'-pyridazinyl)-2H-benzo[b]pyran
143. 6-Trifluormethylthio-2,2-dimethyl-4-(2'-pyrazinyl)-2H-benzo[b]pyran
144. 6-Trifluormethylthio-2,2-dimethyl-4-(2'-pyrazinyl)-2H-benzo[b]pyran-4-ol
145. 6-Trifluormethylthio-2,2-dimethyl-4-(2'-pyrazinyl)-2H-benzo[b]pyran-4'-oxid
146. 6-Trifluormethylthio-2,2-dimethyl-4-(5'-methyl-pyrazinyl)-2H-benzo[b]pyran
147. 6-Trifluormethylthio-2,2-dimethyl-4-(6'-methyl-2'-pyrazinyl)-2H-benzo[b]pyran
148. 6-Trifluormethylthio-2,2-dimethyl-4-(5',6'-dimethyl-2'-pyrazinyl)-2H-benzo[b]pyran
149. 6-Trifluormethoxy-2,2-dimethyl-4-(4'-pyrimidinyl)-3,4-dihydro-2H-benzo[b]pyran
150. 6-Trifluormethylsulfonyl-2,2-dimethyl-4-(5'-pyrimidinyl)-2H-benzo[b]pyran
151. 6-Trifluormethylsulfonyl-2,2-dimethyl-4-(5'-pyrimidinyl)-2H-benzo[b]pyran-4-ol
152. 6-Trifluormethylsulfonyl-2,2-dimethyl-4-(5'-pyrimidinyl)-2H-benzo[b]pyran-1'-oxid
153. 6-Trifluormethylsulfonyl-2,2-dimethyl-4-(3'-chinolinyl)-2H-benzo[b]pyran
154. 6-Trifluormethylsulfonyl-2,2-dimethyl-4-(3'-chinolinolyl) 2H-benzo[b]pyran-1'-oxid
155. 6-Trifluormethylsulfonyl-2,2-dimethyl-4-(4'-isochinolinyl)--2H-benzo[b]pyran
156. 6-Trifluormethylsulfonyl-2,2-dimethyl-4-(4'-isochinolinyl)--2H-benzo[b]pyran-1'-oxid

Die Verbindungen 1-19, 22-27, 44-50, 62-70, 74-77, 97-113, 132, 133, 141, 150, 151 werden besonders bevorzugt, insbesondere 1-19 und 97-113, und ganz besonders werden bevorzugt 1-4, 103 und 107.

Die Verbindungen der Formel I und ihre Salze sowie Säureadditionssalze können dadurch hergestellt werden, daß man
a) vorzugsweise zur Herstellung einer Verbindung der Formel I, in der R₅ eine N-Heteroarylgruppe darstellt, die eine N-Oxidgruppe in der 2-, 3-oder 4-Position trägt, eine Verbindung der allgemeinen Formel I, in der R₁, R₂, R₃, R₄ und R₆ die vorstehend angegebene Bedeutung besitzen und R₅ eine entsprechende N-Heteroarylgruppe mit einem N-Atom in 2-, 3- bzw. 4-Stellung ist, oxidiert, oder
b) zur Herstellung einer der Formel VI entsprechenden Verbindung der Formel I, 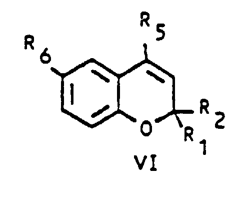 in der R₁, R₂, R₅ und R₆ die oben angegebene Bedeutung haben und R₃ und R₄ zusammen eine Bindung bilden, eine Verbindung der allgemeinen Formel II 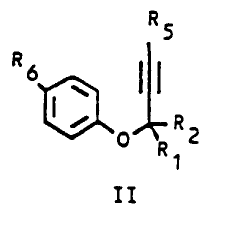 mit der oben angegebenen Bedeutung von R₁, R₂, R₅ und R₆, erhitzt oder aus einer der Formel III entsprechenden Verbindung der Formel I, worin R₃ Wasserstoff und R₄ Hydroxy bedeutet, 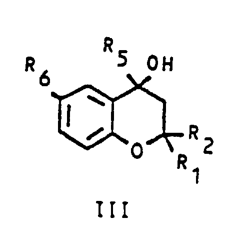 mit der angegebenen Bedeutung von R₁, R₂, R₅ und R₆ Wasser abspaltet, oder
c) zur Herstellung einer der allgemeinen Formel III entsprechenden Verbindung der Formel I mit der oben angegebenen Bedeutung von R₁, R₂, R₅ und R₆, eine Verbindung der allgemeinen Formel IV, 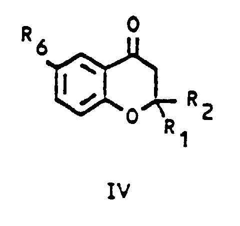 in der R₁, R₂ und R₆ die oben angegebene Bedeutung haben, mit einer metallorganischen Verbindung R₅M umsetzt, in der R₅ die vorstehend angegebene Bedeutung hat und M ein Metall oder Metallhalogenid, vorzugsweise Li-oder MgHal darstellt, wobei Hal Chlor, Brom oder Jod ist, oder
d) zur Herstellung solcher Verbindungen der allgemeinen Formel I, in der R₃ und R₄ Wasserstoff bedeuten und die unter die Formel X der Reaktionsschemata 1 und 2 fallen, in der R₁, R₂, R₅ und R₆ die oben angegebene Bedeutung haben, eine Verbindung der allgemeinen Formel V, 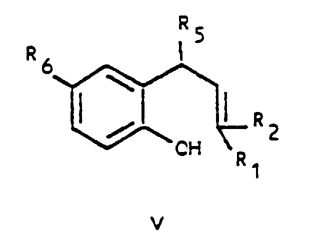 in der R₁, R₂, R₅ und R₆ die oben angegebene Bedeutung haben, cyclisiert, oder eine Verbindung der allgemeinen Formel VI, 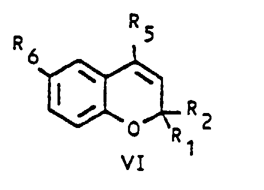 in der R₁, R₂, R₅ und R₆ die angegebene Bedeutung haben, in Gegenwart eines Katalysators hydriert, oder
e) vorzugsweise zur Herstellung einer Verbindung der allgemeinen Formel I, in der R₆ Difluormethylsulfinyl, Difluormethylsulfonyl, Trifluormethylsulfinyl, Trifluormethylsulfonyl, Trifluorethylsulfinyl oder Trifluorethylsulfonyl bedeutet und R₁, R₂, R₃, R₄ und R₅ die vorstehend angegebene Bedeutung haben, eine Verbindung der allgemeinen Formel I, in der R₆ Difluormethylthio, Trifluormethylthio bzw. Trifluorethylthio ist, oxidiert, oder
f) vorzugsweise zur Herstellung einer Verbindung der allgemeinen Formel I, in der R₅ einen N-Heteroaromaten, der in o- oder p-Stellung zum N-Atom durch Chlor oder Cyano substituiert ist, und R₃ und R₄ jeweils Wasserstoff oder zusammen eine Bindung bedeuten und R₁, R₂ und R₆ die oben angegebene Bedeutung haben, eine Verbindung der allgemeinen Formel I, in der R₅ eine N-Heteroaryl-N-Oxidgruppe ist und R₁, R₂, R₃, R₄ und R₆ die entsprechende Bedeutung haben, mit einem Nichtmetallchlorid bzw. einem Silylcyanid umsetzt, oder
g) zur Herstellung einer Verbindung der Formel XII gemäß Schema 1, entsprechend der Formel I, in der R₃ Hydroxy und R₄ Wasserstoff ist, und R₁, R₂, R₅ und R₆ die oben angegebene Bedeutung haben, eine Verbindung der allgemeinen Formel VI, in der R₁, R₂, R₅ und R₆ die oben angegebene Bedeutung haben, zu der Verbindung XI (Schema 1) epoxidiert und anschließend das Epoxid zu der Verbindung der Formel XII (Schema 1) reduziert, oder
h) zur Herstellung von Verbindungen der Formel I, in der R₁, R₂, R₅ und R₆ die oben angegebene Bedeutung haben und R₃ oder R₄ C₁₋₈-Alkoxyl Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeuten, die entsprechenden Verbindungen der Formel I, in der R₃ oder R₄ Hydroxy bedeuten, also die Verbindungen entsprechend den Formeln III und XII (Schema 1), mit entsprechenden Alkylierungsmitteln oder Acylierungsmitteln umsetzt, und gegebenenfalls nach den vorstehenden Verfahren erhaltene Verbindungen in ihre Salze oder Säureadditionssalze oder N-Oxide überführt.

Zur Oxidation eines N-Heteroaromaten R₅ nach Verfahren a) können Reagenzien verwendet werden, die an sich bekannt sind. So kann die Oxidation mit Wasserstoffperoxid, organischen Persäuren wie z. B. Peressigsäure, Perbenzoesäure, m-Chlorperbenzoesäure, Perphthalsäure, ihren Salzen wie z. B. Magnesiumperphthalat, oder mit Natriummetaperiodat, Natriumperborat und dergleichen erfolgen. Dabei wird zweckmäßigerweise in Essigsäure, in einem Alkohol niederer Kettenlänge, bevorzugt in Ethanol oder Methanol, mit oder ohne Zusatz von Wasser, in einem inerten organischen Lösungsmittel wie z. B. Dichlormethan, Dichlorethan, Trichlormethan und bei Temperaturen zwischen 0° C und der Siedetemperatur des Lösungsmittels, bevorzugt bei Raumtemperatur, gearbeitet.

Die Umwandlung einer Verbindung der allgemeinen Formel II gemäß des Verfahrens b) erfolgt bei der Siedetemperatur eines hochsiedenden inerten organischen Lösungsmittels wie z. B. Chlorbenzol, 1,2-Dichlorbenzol, N,N-Diethylanilin, Ethylenglykol, Diphenylether, also bei Temperaturen zwischen ungefähr 120° C und ungefähr 300° C, und auch ohne Lösungsmittel in diesem Temperaturbereich. Wenn mindestens einer der Substitutenten R₁ und R₂ Wasserstoff ist, sind bevorzugt höhere Temperaturen notwendig. Ein erhöhter Druck kann bei dieser Reaktion von Vorteil sein.

Die Dehydratisierung der Verbindungen der allgemeinen Formel III erfolgt nach aus der Literatur bekannten Methoden. Beispielhaft seien genannt: Natriumhydrid in Tetrahydrofuran oder einem anderen inerten organischen Lösungsmittel, vorzugsweise bei der Rückflußtemperatur des Reaktionsgemisches; p-Toluolsulfonsäure in Toluol bei Rückflußtemperatur unter Wasserabscheidung; Umsetzung mit überschüssigem Methansulfonylchlorid/Triethylamin in Dichlormethan oder Chloroform und anschließendem Erhitzen auf Rückflußtemperatur; Erhitzen mit wasserfreiem Kupfer(II)sulfat oder Kaliumhydrogensulfat auf Temperaturen zwischen 50° C und 200° C, vorzugsweise 100-120° C.

Die Addition von metallierten Aromaten bzw. N-Heteroaromaten R₅M an Chromanone der allgemeinen Formel IV gemäß Verfahren c) erfolgt bei Temperaturen zwischen -120° C und Raumtemperatur, vorzugsweise bei zunächst ungefähr -78° C mit anschließender Erwärmung. Die Reaktion erfolgt vorzugsweise in einem Ether wie Diethylether, Tetrahydrofuran, 1,2-Dimethoxyethan, Dioxan. Es können polare Lösungsmittel wie Hexamethylphosphorsäuretriamid oder 1,3-Dimethyl-2-oxohexahydropyrimidin zugesetzt werden.

Die Verfahren zur Erzeugung der Anionen der Aromaten R₅H sind aus der Literatur bekannt. Sie sind entweder aus R₅H durch regiospezifische Deprotonierung mit starken Basen, z. B. Metallalkyl- oder -arylverbindungen, vorzugsweise Verbindungen des Lithiums wie z. B. Methyllithium, Isomere des Butyllithiums, Phenyllithium, Mesityllithium, oder sekundären Metallamiden wie z. B. Lithiumdiisopropylamid (LDA) oder Lithium-2,2,6,6-tetramethylpiperidid (LiTMP), gegebenenfalls unter Zusatz komplexierender, chelatisierender oder der Reaktion ansonsten förderlicher Amin-Verbindungen wie z. B. Tetramethylethylendiamin (TMEDA), Ethylendiamin, Hexamethylphosphorsäuretriamid (HMPT), Polyaminen (DMEU, DMPU, DABCO) erhältlich (Beispiele s.u.a. Tetrahedron 39, 2009 (1983), Organic Reactions 26, 1 (1979), Tetrahedron Letters 29, 773 (1988), Synthesis 1988, 881 und dort zitierte Literatur) oder durch Halogen-Metallaustausch bei den entsprechenden Halogenheterocyclen R₅-Hal, in denen Hal bevorzugt für Brom oder Iod steht, mittels Metallen, Metallalkylen oder Halogenmetallalkylen wie z. B. Lithiumalkylen bzw. Grignardreagenzien nach aus der Literatur bekannten Verfahren zugänglich.

Die Cyclisierung einer Verbindung der allgemeinen Formel V gemäß Verfahren d) kann durch Umsetzen mit einer Säure, vorzugsweise mit einer anorganischen Säure, wie z. B. Schwefelsäure und in einem inerten organischen Lösungsmittel wie einem halogenierten Kohlenwasserstoff, vorzugsweise Dichlormethan, Dichlorethan oder Chloroform, erfolgen. Die Reaktion wird bevorzugt bei Raumtemperatur und vorteilhafterweise in situ durchgeführt.

Die Hydrierung der Verbindungen der allgemeinen Formel VI kann in an sich bekannter Weise in Gegenwart eines Edelmetallkatalysators, insbesondere eines Katalysators mit Metallen der Nebengruppe VIII des Periodensystems, vor allem mit Palladium oder Platin, wie die Metalle selbst oder deren Oxide oder Hydroxide an geeigneten Trägerstoffen wie Aktivkohle unter Wasserstoffdrücken von 1-250 bar und Temperaturen von 20° C bis 200° C in geeigneten inerten organischen Lösungsmitteln wie z. B. Methanol, Ethanol, i-Propanol, Diethylether, Tetrahydrofuran, Dioxan, Essigsäureethylester, Hexan, gegebenenfalls unter Zusatz von Säuren wie Salzsäure oder Essigsäure erfolgen.

Die Oxidation des Thioether-Substituenten R₆ in Verbindungen der allgemeinen Formel I gemäß Verfahren e) kann in bekannter Weise durchgeführt werden. Als Oxidationsmittel können Wasserstoffperoxid, organische Persäuren wie z. B. Peressigsäure, Perbenzoesäure, m-Chlorperbenzoesäure, Perphthalsäure, ihre Salze wie z. B. Magnesiumperphthalat oder anorganische Salze wie z. B. Natriummetaperiodat, Natriumperborat, Kaliumpermanganat oder Oxone^{R} dienen. Als Lösungsmittel finden halogenierte Kohlenwasserstoffe wie Dichlormethan, Chloroform, Dichlorethan, bei Wasserstoffperoxid auch Essigsäure, bei Metallsalzen auch Alkohole niederer Kettenlänge oder Wasser oder Gemische aus beiden Verwendung. Die Umsetzung kann auch in einem zweiphasigen Lösungsmittelsystem unter Phasentransferkatalyse gemäß üblicher Literaturmethoden erfolgen. Zur Oxidation von Thioether-Substituenten R₆ zu Sulfinyl-Substituenten R₆ sind Persäuren, vor allem Peressigsäure und m-Chlorperbenzoesäure, zur Oxidation zu Sulfon-Substituenten R₆ ist Oxone^{R} in Methanol-Wasser-Gemischen bei Temperaturen zwischen 0° C und der Rückflußtemperatur des Gemisches, insbesondere bei Raumtemperatur, bevorzugt. Es können auch Gemische von Sulfinyl- und Sulfonylverbindung anfallen, die durch übliche Methoden wie Kristallisation oder Chromatographie zu trennen sind. Bei diesem Verfahren können auch Verbindungen der Formel I, in denen R₅ eine N-Heteroarylgruppe ist, zu Verbindungen mit R₅ als Substituenten, der eine entsprechende N-Oxidgruppe trägt, oder Verbindungen der Formel I, in denen R₃ und R₄ zusammen eine Bindung bilden, zu einem Benzopyran-3,4-epoxid oxidiert werden. Es kann daher gegebenenfalls von Vorteil sein, die Umwandlung von R₆ in einer der im Reaktionsschema 1 oder 2 aufgeführten Vorstufen vorzunehmen.

Die Umwandlung von N-Oxiden von N-Heteroaryl-Substitutenten R₅ der Formel I gemäß Verfahren f) zu Verbindungen der allgemeinen Formel I mit N-Heteroaryl-Substituenten R₅, die in o- oder p-Stellung zum N-Atom durch Chlor oder Cyano substituiert sind, erfolgt nach aus der Literatur bekannten Methoden. Zur Einführung von Chlor werden vor allem Chloride des Phosphors, bevorzugt Phosphoroxychlorid, verwendet (Chem. Pharm. Bull. 31, 4533(1983); Chem. Pharm. Bull. 36, 2244(1988)). Als Lösungsmittel können chlorierte Kohlenwasserstoffe wie Dichlormethan, Chloroform, 1,2-Dichlorethan, Ether wie Tetrahydrofuran, Dioxan, 1,2-Dimethoxyethan bevorzugt bei der Siedetemperatur des Reaktionsgemisches dienen. Die Reaktion kann auch ohne ein Lösungsmittel, also mit einem Überschuß an Chlorierungsmittel, bei Temperaturen zwischen 50° C und 150° C, bevorzugt bei ungefähr 110° C, durchgeführt, und das in der Regel entstehende Gemisch aus o- und p-Chlorverbindung kann chromatographisch aufgetrennt werden. Zur Einführung von Cyano dient bevorzugt Trimethylsilylcyanid (J. Org. Chem. 48, 1375(1983); Synthesis 1983, 316; Synthesis 1984, 681; Chem. Pharm. Bull. 33, 565(1985); Chem. Pharm. Bull. 35, 3119(1987)), das auch in situ aus Trimethylchlorsilan und Natrium- oder Kaliumcyanid während der Reaktion erzeugt werden kann, unter Zusatz eines tertiären Amins als Base, z. B. Triethylamin. Als Lösungsmittel werden chlorierte Kohlenwasserstoffe, z. B. Dichlormethan, Dichlorethan, Chloroform, oder Dimethylformamid oder bevorzugt Acetonitril bei Reaktionstemperaturen zwischen 0° C und der Siedetemperatur des Reaktionsgemisches verwendet.

Die Reduktion eines Benzopyran-3,4-epoxids gemäß Verfahren g) kann mit verschiedenen Reduktionsmitteln erfolgen, z. B. durch eine Hydrierung, die bevorzugt mit den gleichen Edelmetallkatalysatoren und unter ähnlichen Reaktionsbedingungen durchgeführt wird, wie bereits für die Verfahrensvariante d) beschrieben. Möglich ist auch die Umsetzung mit Phenylselenid, das aus Diphenyldiselenid durch Reduktion in einem Alkohol niederer Kettenlänge, z. B. Ethanol, erzeugt und in situ umgesetzt wird (Tetrahedron Letters 28, 4293(1987)). Als weitere Reagenzien kommen in Frage: ein Überschuß an Alkalimetalliodid in gepufferter essigsaurer acetonischer Lösung, vorzugsweise bei Raumtemperatur (Chem. Ber. 109, 3907(1976)), Übergangsmetallverbindungen niedriger Oxidationsstufen, bevorzugt Samarium(II)iodid in Tetrahydrofuran (J. Org. Chem. 51, 2596(1986); Tetrahedron Letters 28, 4437(1987)) und mit geringerer Selektivität, vor allem dann, wenn weitere reduzierbare Gruppen, z. B. eine N-Oxidgruppe, im Molekül vorhanden sind, auch mit komplexen Metallhydriden wie Lithiumaluminiumhydrid, Natriumborhydrid, Natriumcyanoborhydrid, Natriumbis(2-methoxyethoxy)aluminiumhydrid.

Die Verbindungen der allgemeinen Formel I, in denen R₃ oder R₄ Hydroxy ist, die also nach den Verfahren g) bzw. c) erhältlich sind, können entsprechend Verfahren h) durch Umwandlung der freien Hydroxygruppe zu weiteren Verbindungen gemäß Formel I umgesetzt werden, und zwar durch Alkylierung zu Ethern, durch Acylierung zu Carbonsäureestern, Carbonaten oder Carbamaten. Die dazu verwendeten Methoden sind Standardmethoden der organischen Synthese.

Eine Alkylierung kann z. B. durch Verwendung eines C₁₋₆-Alkyliodids in einem inerten Solvens wie Toluol oder Dimethylformamid in Anwesenheit einer Base wie Kaliumhydroxid oder Bariumoxid ausgeführt werden.

Eine Veresterung kann durch Verwendung eines C₁₋₈-Acylchlorids oder Acylanhydrids Oder eines anderen aktivierten Derivats der betreffenden Alkansäure, gegebenenfalls unter Anwesenheit einer organischen Base wie Pyridin oder Triethylamin oder einer anorganischen Base wie Kaliumcarbonat, gegebenenfalls unter Mitwirkung von Katalysatoren wie 4-(N,N-Dimethylamino)-pyridin oder mit der C₁₋₈-Carbonsäure unter Mitwirkung von Kondensationsreagentien wie Dicyclohexylcarbodiimid in einem inerten Solvens, gegebenenfalls bei erhöhter Temperatur durchgeführt werden.

Eine Umsetzung zu Carbonaten erfolgt analog durch Reaktion mit Chlorameisensäure-C₁₋₈-alkylestern unter oben angegebenen Bedingungen.

Die Umsetzung zu Carbamaten erfolgt entweder in Analogie zu oben beschriebenen Verfahren durch Reaktion mit Mono- oder Dialkylaminocarbamoylchloriden oder durch Umsetzung mit C₁₋₈-Alkylisocyanaten in einem inerten Solvens, z. B. Toluol, bei Temperaturen zwischen 0° C und der Siedetemperatur des Reaktionsgemisches. Durch Umsetzung mit Phosgen und nachfolgender Hydrolyse des entsprechenden Chlorameisensäureesters oder duch Umsetzung mit Ameisensäure in Pyridin läßt sich Formyloxy einführen.

Verbindungen der allgemeinen Formel I, in denen R₅ ein Substituent der Untergruppe A) ist, also eine Aryl- oder N-Heteroarylgruppe mit einer Hydroxygruppe in 2-Stellung, sind aus Verbindungen erhältlich, in denen diese durch für phenolische Hydroxygruppen übliche Schutzgruppen geschützt sind. Bevorzugt sind C₁₋₈-Alkoxygruppen, besonders bevorzugt Methoxy, oder Formaldehydacetale wie z. B. Methoxymethyl.

Die Spaltung einer Ethergruppe wird in an sich bekannter Weise durchgeführt. Die Umsetzung kann beispielsweise mit einem Alkalialkanthiolat niederer Kettenlänge, vorzugsweise Natriummethanthiolat, zweckmäßigerweise in einem inerten organischen Lösungsmittel wie z. B. Dimethylformamid bei erhöhter Temperatur, z. B. bei etwa 100° C, aber auch unter Verwendung anderer Reagenzien wie z. B. Lithiumiodid, einem Silylhalogenid oder einem Borhalogenid erfolgen.

Die Spaltung eines Formaldehydacetals kann nach aus der Literatur üblichen Methoden erfolgen, z. B. unter sauren Reaktionsbedingungen, insbesondere mit Mineralsäuren wie z. B. Salzsäure.

Die für diese beschriebenen Hydrolysen geeigneten Vorstufen sind nach den vorstehend aufgeführten Verfahrensvarianten, insbesondere b), c) und d), besonders bevorzugt c), erhältlich. Zur Metallierung von Alkoxy-N-Heteroaromaten sind viele Beispiele aus der Literatur bekannt (s. Erläuterung der Verfahrensvariante c)). Ortho-Metallierungen von Anisol-Derivaten (J. Org. Chem. 41, 3653(1976)) und von Methoxymethoxybenzol-Derivaten (Tetrahedron Lett. 22, 3923(1981); J. Org. Chem. 47, 2101(1982)) sind ebenfalls bekannt.

Speziell wenn R₅ ein N-Heteroaromat ist mit einem N-Atom in der 3-Stellung und einer Hydroxygruppe in der 2-Stellung, so ist eine solche Verbindung aus einer Verbindung der allgemeinen Formel I erhältlich, in der R₅ ein Heteroaromat-N-oxid ist mit dem Stickstoff in der gleichen Position. Die Umwandlung wird durchgeführt mit einem C₁₋₈-Carbonsäureanhydrid, bevorzugt Essigsäureanhydrid, und anschließender Hydrolyse. Sie erfolgt bei erhöhter Temperatur, vorzugsweise bei der Siedetemperatur des Reaktionsgemisches. Die Hydrolyse des erhaltenen Produkts, nämlich einer Verbindung der Formel I, in der R₅ eine N-Heteroarylgruppe mit einem N-Atom in 3-Stellung und einer Alkanoyloxygruppe in 2-Stellung mit dem Alkanoylrest des C₁₋₈-Carbonsäureanhydrids bedeutet, kann sauer oder basisch nach bekannten Verfahren erfolgen. Zur Säurehydrolyse finden wäßrige Mineralsäuren, wie z. B. Salzsäure, Bromwasserstoffsäure, Schwefelsäure oder organische Säuren, wie z. B. p-Toluolsulfonsäure, zweckmäßigerweise in einem inerten organischen Lösungsmittel, wie z. B. Dioxan, Tetrahydrofuran, Verwendung. Die Reaktion findet vorzugsweise bei Raumtemperatur statt. Die basische Hydrolyse kann mit einem Alkalimetallhydroxid, z. B. Natriumhydroxid, oder einem Alkalimetall-C₁₋₈-alkoxid, z. B. Natriummethoxid, Natriumethoxid, vorzugsweise in einem inerten organischen Lösungsmittel wie Methanol oder Ethanol bei Raumtemperatur durchgeführt werden.

Die Synthese der vorstehend genannten 2-Hydroxy-N-Heteroaromaten aus N-Oxiden von 3-N-Heteroaromaten kann auch über die nach Verfahren f) beschriebenen 2-Halogen-3-N-Heteroaromaten erfolgen, deren Hydrolyse im sauren sowie im basischen Reaktionsmedium wie ebenfalls vorstehend beschrieben durchgeführt werden kann.

Es können einige Substituenten an den Aromaten und N-Heteroaromaten R₅ in funktionell andere nach allgemein üblichen und bekannten Verfahren übergeführt werden. So läßt sich z. B. eine Nitrogruppe zu einer Aminogruppe reduzieren. Als Reduktionsmittel dient bevorzugt Eisenpulver/Essigsäure. Eine Aminogruppe kann nach üblichen Verfahren einer Sandmeyer-Reaktion in eine Cyanogruppe, ein Halogen oder eine Hydroxygruppe übergeführt werden. Weiter kann eine Cyanogruppe zu einer Carboxygruppe hydrolysiert und diese z. B. mit einem Diazoalkan zu einem Carbonsäureester, bevorzugt mit Diazomethan zu einem Carbonsäuremethylester, umgewandelt werden. Andere Carbonsäure-Derivate sind aus der Cyanogruppe ebenfalls nach bekannten Verfahren erhältlich.

Ein Halogenatom, bevorzugt ein Chloratom, kann in eine C₁₋₈-Alkoxy-, C₁₋₈-Monoalkylamino- oder C₁₋₈-Dialkylaminogruppe übergeführt werden. Der Austausch kann in an sich bekannter Weise mit einem Alkalimetallalkoxid der entsprechenden Kettenlänge, z. B. mit Natriummethoxid oder mit Natriumethoxid, erfolgen. Die Reaktion wird bevorzugt in einem inerten organischen Lösungsmittel wie dem Alkohol, der dem Alkalimetallalkoxid entspricht, durchgeführt, vorzugsweise unter der Rückflußtemperatur des Reaktionsgemisches.

Der Austausch mit einem Alkalimetallamid wird in ähnlicher Weise durchgeführt, wobei jedoch zweckmäßigerweise ein Ether, z. B. Tetrahydrofuran, Dioxan oder 1,2-Dimethoxyethan, als inertes organisches Lösungsmittel dient. Die Alkalimetallamide werden vorzugsweise aus den entsprechenden Aminen mit Alkalimetallalkylen, z. B. Butyllithium, in dem gleichen Lösungsmittel erzeugt und in situ umgesetzt.

Die Auftrennung von Diastereomeren und cis/trans-Gemischen kann nach allgemein üblichen Methoden erfolgen, z. B. durch Chromatographie oder Kristallisation.

Verbindungen der allgemeinen Formel I, die als Racemate anfallen, oder gewisse racemische Vorstufen davon können nach bekannten Methoden in ihre optischen Antipoden aufgetrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz oder eine andere Verbindung bildet, Trennung der Diastereoisomeren durch fraktionierte Kristallisation und Freisetzung der Enantiomeren durch geeignete Mittel. Besonders geeignete optisch aktive Säuren sind beispielsweise die d- und 1-Formen der Weinsäure, Ditoluylweinsäure, Äpfelsäure, Mandelsäure, Camphersulfonsäure oder Pyrrolidon-carbonsäure. Geeignete optisch aktive Basen sind alpha-Phenylethylamin, Menthylamin, Ephedrin, Brucin und Chinin.

Bevorzugt wird eine chirale Verbindung der allgemeinen Formel I, in der R₅ ein basischer N-Heteroaromat ist, mit einer der oben aufgeführten chiralen Säuren umgesetzt oder eine chirale Verbindung der allgemeinen Formel I, die eine freie Hydroxygruppe enthält, insbesondere wenn R₃ oder R₄ Hydroxy ist, mit einer chiralen Säure verestert.

Vorteilhafterweise wird der aktivere der Antipoden isoliert. Gemäß der Erfindung ist es jedoch auch möglich, die reinen Enantiomeren durch asymmetrische Synthese zu erhalten.

Verbindungen der allgemeinen Formel I, in denen R₅ ein N-Heteroaromat ist oder eine Aminogruppe enthält, sind basisch und können daher in ihre Säureadditionssalze übergeführt werden. Verbindungen der allgemeinen Formel I, in denen R₅ eine phenolische Hydroxygruppe oder eine Carboxylgruppe enthalten, sind sauer und können daher in ihre Salze übergeführt werden.

Bevorzugt werden physiologisch verträgliche Salze oder Säureadditionssalze. Hierfür sind als anorganische Säuren beispielsweise Schwefelsäure oder Halogenwasserstoffsäuren, zum Beispiel Salzsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium- oder Kaliumhydroxid erhalten werden.

Die zur Darstellung von Verbindungen der allgemeinen Formel I notwendigen Vor- und Zwischenprodukte, die, sofern sie neu, auch Gegenstand der vorliegenden Erfindung, sind in den Reaktionsschemata 1 und 2 aufgeführt. In allen Fällen wird von den Phenolen VII ausgegangen, in denen R₆ die oben angegebene Bedeutung hat. Sie sind bekannt oder können nach bekannten Verfahren hergestellt werden, z. B. durch Reduktion der entsprechenden 4-substituierten Nitroaromaten, beispielsweise mit Wasserstoff und Raneynickel als Katalysator oder durch nascierenden Wasserstoff zu den entsprechenden 4-substituierten Anilinen und Diazotierung und Verkochung derselben zu den besagten 4-substituierten Phenolen.

In Fällen, in denen R₆ die Bedeutung Difluormethylthio, Difluormethylsulfinyl, Difluormethylsulfonyl, Trifluormethylthio, Trifluormethylsulfinyl, Trifluormethylsulfonyl, 2,2,2-Trifluorethylthio, 2,2,2-Trifluorethylsulfinyl und 2,2,2-Trifluorethylsulfonyl hat, können die genannten Reste häufig vorteilhafter durch chemische Transformationen von Zwischenprodukten, in denen R₆ eine andere als die obengenannte Bedeutung hat, eingeführt werden. So werden z. B. 2H-Benzo[b]pyrane der allgemeinen Formel VI, in 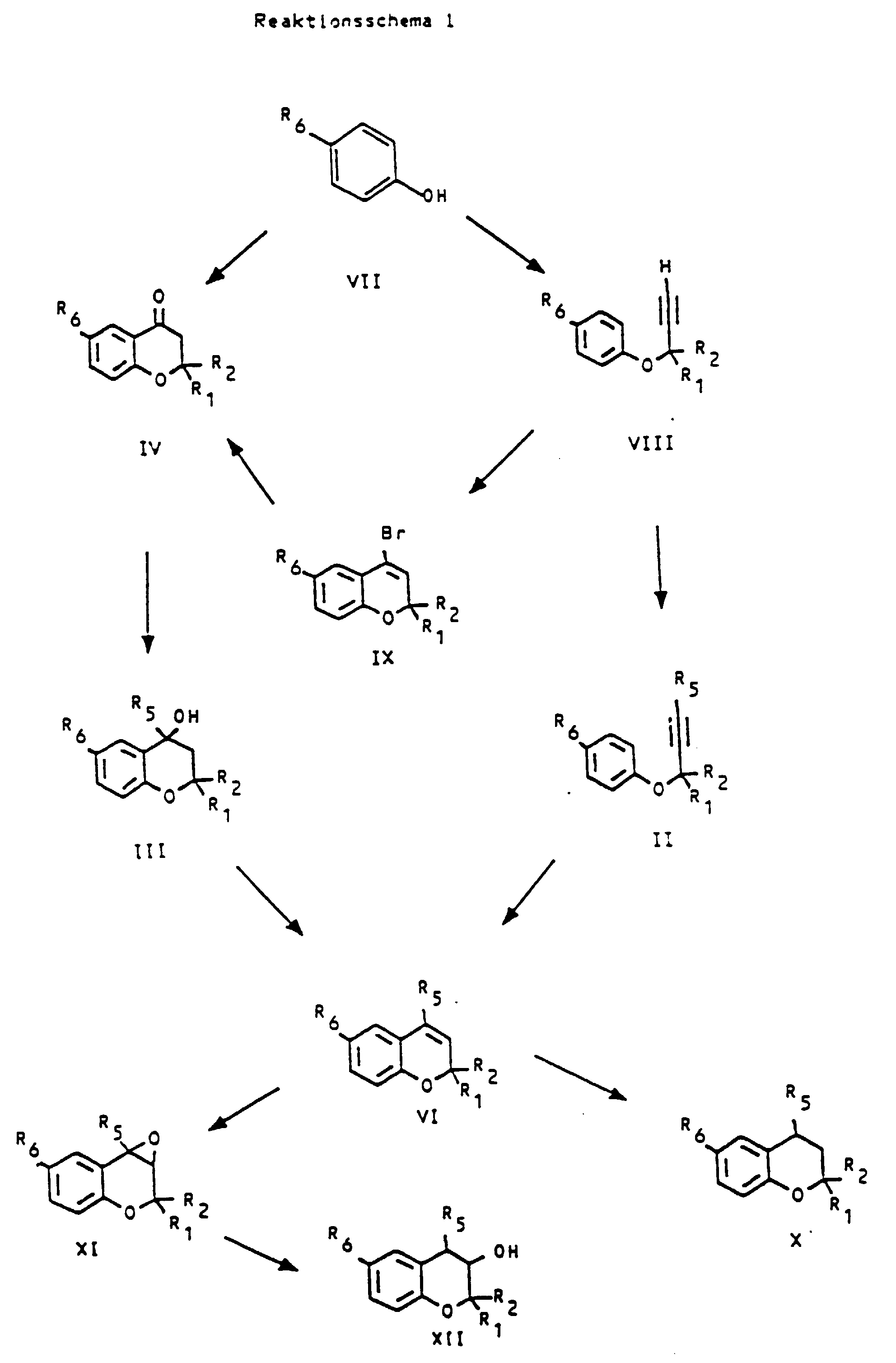 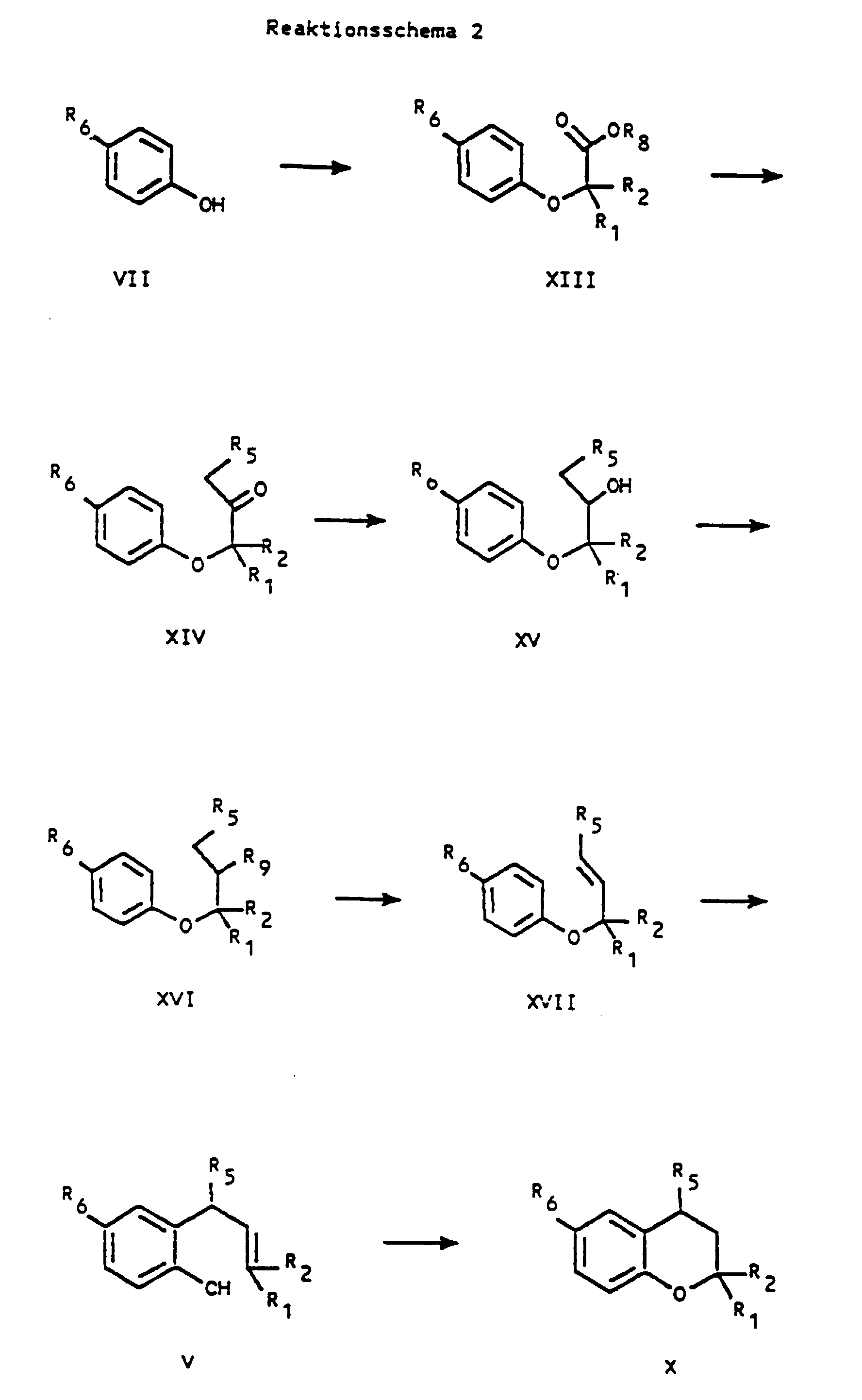 denen R₆ die Bedeutung Difluormethylsulfonyl, Trifluormethylsulfonyl und 2,2,2-Trifluorethylsulfonyl hat, durch Umsetzung der entsprechenden Fluoralkylsulfonylfluoride mit 2H-Benzo[b]pyranen der allgemeinen Formel VI, in denen R₆ die Bedeutung MgHal hat, wobei Hal die Bedeutung Chlor, Iod und insbesondere Brom hat, erhalten, sofern R₅ keine Substituenten enthält, die diese Umsetzung stören, also R₅ unsubstituiert oder z. B. durch C₁₋₈-Alkyl, Phenyl oder C₁₋₈-Alkoxy substituiert ist. Ebenso ist es möglich, die oben beschriebenen Grignard-Verbindungen von 2H-Benzo[b]pyranen mit Disulfiden der allgemeinen Formel R₇-S-S-R₇, in denen R₇ die Bedeutung Trifluormethyl, Difluormethyl und 2,2,2,-Trifluorethyl hat, zu 2H-Benzo[b]pyranen, in denen R₆ die Bedeutung Difluormethylthio, Trifluormethylthio und 2,2,2,-Trifluorethylthio hat, umzusetzen.

Weiterhin ist es möglich, ausgehend von Zwischen- oder Endprodukten, in denen R₆ ein Schwefelatom enthält, nach an sich üblichen Methoden durch Reduktion und insbesondere Oxidation zu Zwischen- und Endprodukten, in denen das bezeichnete Schwefelatom eine andere Oxidationsstufe aufweist, zu gelangen. Mögliche Oxidationsmittel wurden bereits bei der Beschreibung der Verfahrensvariante e) aufgeführt. So kann z. B. 4-Difluormethylsulfonylphenol, 4-Trifluormethylsulfonylphenol oder 4-(2,2,2-Trifluorethylsulfonyl)phenol günstiger als nach bekannten Verfahren durch Oxidation der entsprechenden Fluoralkylthiophenole mit Oxone^{R} in Methanol-Wasser-Gemischen bei Temperaturen zwischen -10° C und der Rückflußtemperatur des Reaktionsgemisches, vorzugsweise bei Temperaturen zwischen 0° C und 25° C erhalten werden.

Insbesondere in Fällen, in denen R₆ die Bedeutung Difluormethylsulfinyl, Trifluormethylsulfinyl oder 2,2,2-Trifluorethylsulfinyl hat, ist es günstiger, ausgehend von den entsprechenden 4-Fluoralkylthiophenolen gemäß Reaktionsschema 1 zunächst die entsprechenden 6-Fluoralkylthio-2H-benzo[b]-pyrane VI, bei denen R₁, R₂ und R₅ die oben angegebene Bedeutung haben, herzustellen und anschließend die gewünschte Oxidation zu den jeweiligen 6-Fluoralkylsulfinyl-2H-benzo[b]pyranen der allgemeinen Formel VI vorzunehmen.

In manchen Fällen ist es möglich, C₁₋₈-Dialkoxyphosphoryl als Substituenten R₆ in Zwischen- und Endprodukte der vorliegenden Erfindung einzuführen, indem man entsprechende Verbindungen, in denen R₆ Brom ist, mit einem C₁₋₈-Trialkylphosphit in Gegenwart eines Nickel(II)halogenids, vorzugsweise Nickel(II)chlorid, bei erhöhter Temperatur, vorzugsweise bei ungefähr 180° C, umsetzt. Zwischen- und Endprodukte, in denen R₆ Phosphono bedeutet, können nach üblichen Methoden der Spaltung von Phosphonsäureestern aus den entsprechenden C₁₋₈-Dialkoxyphosphoryl-Verbindungen erhalten werden. Genauso kann auch die umgekehrte Transformation zu Phosphonsäureestern durchgeführt werden.

Verbindungen der allgemeinen Formel VIII sind aus den Phenolen VII durch Umsetzung mit Verbindungen der allgemeinen Formel Y-CR¹R²-C≡CH, worin Y Chlor, Brom oder Hydroxy bedeutet, erhältlich. Wenn Y Chlor oder Brom ist, erfolgt die Reaktion in bekannter und mehrfach beschriebener Weise (z. B. J. Org. Chem. 38, 3832(1973); J. Org. Chem. 39, 881(1974); J. Org. Chem. 37, 841(1972); J. Med. Chem. 26, 1582(1983)) in einem inerten organischen Lösungsmittel in Gegenwart einer Base. Bevorzugte Reaktionsbedingungen sind z. B. Kaliumcarbonat als Base in Dimethylformamid bei etwa 90° C oder bevorzugt in Aceton oder Butanon unter Rückfluß, Natrium- oder Kaliumhydroxid mit einem Phasentransferkatalysator wie z. B. Trimethylbenzylammoniumhydroxid in Methanol bei Raumtemperatur. Wenn Y Hydroxy ist, kann die Reaktion in Gegenwart eines Kondensationsmittels, z. B. Diethylazodicarboxylat/Triphenylphosphin (s. Synthesis 1981, 1), in einem inerten organischen Lösungsmittel, z. B. Dichlormethan, durchgeführt werden.

Eine Verbindung der allgemeinen Formel II kann aus einer Verbindung der allgemeinen Formel VIII erhalten werden, indem man diese mit einem halogenierten Aromaten oder N-Heteroaromaten R₅Hal, in dem Halogen Brom oder Iod, bevorzugt Iod, bedeutet, umsetzt. Die Reaktion erfolgt in Gegenwart von Kupfer(I)iodid und einer Palladiumverbindung als Katalysator, bevorzugt Bis(triphenylphosphin)palladiumdichlorid oder -diacetat, und einem Triarylphosphin, bevorzugt Triphenylphosphin, in einem aliphatischen Amin niederer Kettenlänge, bevorzugt in Triethylamin oder Diethylamin, als Lösungsmittel bei Temperaturen zwischen der Raumtemperatur und dem Siedepunkt des Reaktionsgemisches, bevorzugt bei ungefähr 80° C. Es kann von Vorteil sein, die Reaktion in einem abgeschlossenen Reaktionsgefäß, also bei etwas mehr als Atmosphärendruck ablaufen zu lassen. Derartige Reaktionen sind aus der Literatur in großer Zahl bekannt (z. B. Heterocycles 9, 271(1978); Synthesis 1980, 627; Synthesis 1981, 364; Chem. Pharm. Bull. 28, 3488(1980); Synthesis 1983, 313; J. Org. Chem. 53, 386(1988)).

Die Benzo[b]pyran-4-one der allgemeinen Formel IV können nach bekannten Literaturverfahren (Angew. Chem. 94, 254(1982)) dargestellt werden, bevorzugt durch Kondensation von Ketonen bzw. Aldehyden der allgemeinen Formel R₁R₂C=O mit Phenolen der allgemeinen Formel VII, die zusätzlich in der 2-Stellung durch eine Acetylgruppe substituiert sind, oder durch Hydrolyse der 4-Brombenzo[b]pyrane IX. Diese wird mit starken anorganischen Säuren, z. B. mit konzentrierter Schwefelsäure vorzugsweise bei Raumtemperatur, oder mit Schwermetallsalzen in organischen Säuren, bevorzugt mit Salzen des Quecksilbers wie z. B. Quecksilber(II)acetat oder Quecksilber(II)trifluoracetat in Eisessig bzw. Trifluoressigsäure, aber auch in anderen inerten organischen Lösungsmitteln wie z. B. Acetonitril, Nitromethan, Dichlormethan bei Temperaturen zwischen 0° C und der Rückflußtemperatur des Reaktionsgemisches durchgeführt. Ein Zusatz einer Lewis-Säure, z. B. Bortrifluorid, kann von Vorteil sein (Tetrahedron Letters 1978, 1943; ibid. 1979, 3489).

4-Brombenzo[b]pyrane IX sind erhältlich, indem man zunächst die Propargylether der allgemeinen Formel VIII unter den für das Verfahren b) beschriebenen Reaktionsbedingungen zu Benzo[b]pyranen cyclisiert, die in den Positionen 3 und 4 Wasserstoff und in den Positionen 2 und 6 die Substituenten R₁, R₂ und R₆ mit der oben angegebenen Bedeutung tragen, und anschließend an die C-C-Doppelbindung zwischen dem C-3 und dem C-4 des Benzopyrans Brom addiert, bevorzugt in einem inerten organischen Lösungsmittel wie Tetrachlormethan oder Chloroform bei Temperaturen zwischen 0° C und Raumtemperatur. Mit Basen wird aus den erhaltenen trans-3,4-Dibrombenzo[b]pyranen ausschließlich oder bevorzugt das 3-Brom zu den Verbindungen der allgemeinen Formel IX abgespalten. Vorzugsweise werden Alkalimetallalkoxide wie z. B. Natriummethanolat, Natriumethanolat, Kalium-tert.-butanolat in dem entsprechenden Alkohol als Lösungsmittel, tertiäre Amine wie z. B. Triethylamin, Diisopropylethylamin, Diazabicycloundecan (DBU) oder Alkalimetallhydride wie z. B. Natriumhydrid in inerten organischen Lösungsmitteln wie Diethylether, Tetrahydrofuran, 1,2-Dimethoxyethan, Dichlormethan und dergleichen als Basen verwendet. Die Reaktionstemperatur ist bevorzugt die Raumtemperatur oder eine leicht erhöhte.

Benzo[b]pyran-3,4-epoxide der allgemeinen Formel XI können aus den Benzo[b]pyranen VI durch Oxidation nach bekannten Verfahren dargestellt werden. Ist R₅ ein N-Heteroaromat und/oder R₆ Difluormethylthio, Difluormethylsulfinyl, Trifluormethylthio, Trifluormethylsulfinyl, Trifluorethylthio oder Trifluorethylsulfinyl, so kann als Nebenreaktion auch am Stickstoff bzw. am Schwefel eine Oxidation eintreten. Als Reagenzien können Persäuren, z. B. Peressigsäure, Perbenzoesäure, Perphthalsäure, m-Chlorperbenzoesäure in inerten wasserfreien organischen Lösungsmitteln wie Dichlormethan, Chloroform, Dichlorethan, Diethylether, Tetrahydrofuran oder Acetonitril, oder Magnesiumperphthalat in einem Alkohol niederer Kettenlänge mit oder ohne Zusatz von Wasser oder Wasserstoffperoxid in Gegenwart eines Wolframats, bevorzugt Natriumwolframat, in dem gleichen Lösungsmittel bei Temperaturen zwischen Raumtemperatur und der Rückflußtemperatur des Reaktionsgemisches Verwendung finden.

Der Syntheseweg zu Verbindungen der allgemeinen Formel V durch Cyclisierung nach Verfahren d) ist im Reaktionsschema 2 dargestellt. Phenole der allgemeinen Formel VII werden mit α-Halogencarbonsäureestern HalR₁R₂CCOOR₈, in denen Halogen bevorzugt für Brom oder Chlor, R8 für einen Alkylrest niederer Kettenlänge, bevorzugt für Methyl oder Ethyl, steht, nach bekannten Verfahren zu Verbindungen der allgemeinen Formel XIII umgesetzt. Als Base dient dabei ein Alkalialkoxid des Alkohols R₈OH in demselben als Lösungsmittel, ein Alkalimetallhydrid, z. B. Natriumhydrid, in einem Ether, z. B. Tetrahydrofuran, oder in Dimethylformamid, oder ein Alkalimetallcarbonat, z. B. Kaliumcarbonat, in Aceton oder Butanon als Lösungsmittel. Die Reaktion wird bei Temperaturen zwischen 0° C und der Siedetemperatur des Reaktionsgemisches, bevorzugt bei Raumtemperatur, durchgeführt.

Die Umsetzung der Verbindungen der allgemeinen Formel XIII zu den Ketonen der allgemeinen Formel XIV wird mit Anionen von Methyl-Heterocyclen MeR₅, in denen R₅ bevorzugt ein N-Heterocyclus mit einem N-Atom oder einer N-Oxidgruppe in 2-Stellung ist, z. B. 4-Methylpyrimidin oder 2-Picolin-N-oxid, durchgeführt. Die Anionen werden aus den Heterocyclen MeR₅ mit starken Basen, z. B. mit Alkalimetalldialkylamiden niederer Kettenlänge, z. B. Lithiumdiisopropylamid, oder mit Alkalimetallalkylen wie Butyllithium in inerten organischen Lösungsmitteln wie Diethylether oder Tetrahydrofuran erzeugt. Die Umsetzung erfolgt vorzugsweise in dem gleichen Lösungsmittel bei Temperaturen zwischen -78° C und der Rückflußtemperatur des Reaktionsgemisches, bevorzugt bei tieferer Temperatur mit anschließender Erwärmung auf Raumtemperatur.

Die Reduktion der Ketone XIV zu den Alkoholen der allgemeinen Formel XV kann in bekannter Weise, bevorzugt mit komplexen Hydriden, z. B. Natriumborhydrid in einem Alkohol wie Methanol oder Ethanol, oder mit Lithiumaluminiumhydrid in Diethylether oder Tetrahydrofuran als Lösungsmittel, vorzugsweise bei Raumtemperatur durchgeführt werden. Soll gegebenenfalls ein N-Heterocyclus R₅ in sein N-Oxid übergeführt werden, so geschieht dies bevorzugt auf dieser Stufe der Synthesesequenz nach Methoden und Reaktionsbedingungen, die unter Verfahren a) beschrieben wurden.

Die Umwandlung der Alkohole XV in Verbindungen der allgemeinen Formel XVI, in denen R₉ eine Abgangsgruppe ist, wird nach bekannten Verfahren durchgeführt. R₉ ist vorzugsweise Iod, ein Alkansulfonat, z. B. Methansulfonat, oder ein Arylsulfonat, z. B. Phenylsulfonat oder p-Tolylsulfonat. Die Sulfonate werden mit den entsprechenden Sulfonsäurechloriden in Gegenwart eines säurebindenden Mittels, z. B. eines tertiären Amins wie Triethylamin, Diisopropylethylamin oder Pyridin, das auch als Lösungsmittel verwendet werden kann, erhalten. Die Reaktion wird ansonsten in einem üblichen inerten organischen Lösungsmittel vorzugsweise bei Raumtemperatur durchgeführt. Verbindungen der allgemeinen Formel XVI, in denen R₉ Iod ist, sind aus den vorstehend beschriebenen Sulfonaten der allgemeinen Formel XVI nach bekannten Verfahren mit einem Alkalimetalliodid, z. B. Natriumiodid, erhältlich. Die Umsetzung erfolgt in einem inerten organischen Lösungsmittel, bevorzugt Aceton oder Butanon, bei erhöhter Temperatur, vorzugsweise bei der Rückflußtemperatur des Reaktionsgemisches.

Die Verbindungen der allgemeinen Formel XVI werden in Verbindungen der allgemeinen Formel V übergeführt, wobei die Stufe der Olefine XVII durchlaufen wird. Diese können isoliert werden, wenn die Abspaltung von HR₉ bei Raumtemperatur oder leicht erhöhter durchgeführt wird. Zur Reaktion werden starke Basen wie z. B. Natriumhydrid, Natriummethanolat, Natriumethanolat, Kalium-tert.-butanolat oder tertiäre Amine wie Diisopropylethylamin, Triethylamin, Diazabicycloundecan (DBU) in inerten organischen Lösungsmitteln wie Tetrahydrofuran, Diethylether, Methanol, Ethanol und dergleichen verwendet. Die Umsetzung der Verbindungen XVII erfolgt dann durch Erhitzen in hochsiedenden aromatischen Kohlenwasserstoffen wie Chlorbenzol oder 1,2-Dichlorbenzol. Die direkte Umwandlung von Verbindungen XVI in Verbindungen der Formel V erfolgt bevorzugt unter der Rückflußtemperatur des obigen Reaktionsgemisches mit tertiären Aminen als Base. Verbindungen der Formel XVII können auch bereits als Nebenprodukte aus den Alkoholen XV bei der Darstellung der Verbindungen der allgemeinen Formel XVI auftreten.

Die erfindungsgemäßen Verbindungen der Formel I, ihre physiologisch verträglichen Salze und Säureadditionssalze sowie deren tautomere und optische Isomere sind therapeutische Wirkstoffe, besitzen hohe pharmakologische Wirkung und sind wertvolle Arzneimittel. Insbesondere zeigen sie vasodilatierende, gefäßspasmolytische, insbesondere broncholytische Wirkung, wobei sich die gefäßspasmolytische Wirkung im gesamten Gefäßsystem oder auch mehr oder weniger isoliert in umschriebenen Gefäßgebieten wie Gehirn-, Coronar- oder Peripheriegefäßen entfalten kann.

Die erfindungsgemäßen Verbindungen haben insbesondere blutdrucksenkende Wirkung und können somit als antihypertensive Mittel verwendet werden.

Die erfindungsgemäßen Substanzen zeichnen sich durch eine beträchtliche Senkung des arteriellen Blutdrucks aus. Dosen von 0.01 - 10 mg/kg p.o. führten zu einer Senkung des Blutdrucks um mindestens 20 % an hypertensiven Ratten.

Die erfindungsgemäßen Substanzen zeichnen sich durch eine besondere Beeinflussung der Kaliumionenströmung in den Zellen aus. Insbesondere sind sie Kaliumkanalaktivatoren. Sie eignen sich zur Prophylaxe und zur Behandlung folgender Erkrankungen bei Warmblütern, insbesondere des Menschen:
1. Bluthochdruck, insbesondere arterieller Bluthochdruck,
2. Herzinsuffizienz, Coronar-Insuffizienz, Angina Pectoris,
3. arterielle Verschlußkrankheit und periphere Durchblutungsstörungen,
4. cerebrale Insuffizienz, Migräne, Schwindel, Erkrankungen des Innenohrs beziehungsweise des Gehörapparats,
5. erhöhter Augeninnendruck, Glaukom, Sehschwäche,
6. Niereninsuffizienz, organische Erkrankungen der harnableitenden Wege sowie der Akzessorischen Drüsen der Harnwege, Potenzstörungen,
7. organische Störungen des Magendarmtraktes sowie der Bauchspeicheldrüse und Leber,
8. mangelnde Durchblutung der Kopfhaut, Haarausfall,
9. Erkrankungen der Luftwege einschließlich Asthmabronchiale,
10. metabolische Erkrankungen,
11. spasmogene Erkrankungen des Uterus,
12. Inkontinenz.

Weiterhin fördern die erfindungsgemäßen Verbindungen die Durchblutung der Kopfhaut und den Haarwuchs. Sie sind auch tokolytisch wirksam.

Die erfindungsgemäßen Verbindungen zeigen eine lange Wirkdauer bei nur geringer Toxizität. Daher eignen sie sich insbesondere zur Behandlung akuter und chronischer Herzkrankheiten, zur Therapie des Bluthochdrucks, der Herzinsuffizienz sowie zur Behandlung von Asthma und cerebralen und peripheren Durchblutungsstörungen.

Die Verbindungen der vorliegenden Erfindung können beim Menschen oral oder parenteral in einer Dosierung von 0,001 bis 100 mg, vorzugsweise 0,01 bis 50 mg, besonders bevorzugt 0,05 bis 10 mg pro Tag, angewendet werden, insbesondere auch in unterteilten Dosen, zum Beispiel zweimal bis viermal täglich. Diese Dosierungen sind vorteilhaft für die Behandlung der vorstehend genannten Krankheiten, insbesondere von Herzkrankheiten, Hypertonie, Asthma und Durchblutungsstörungen.

Im allgemeinen hat es sich als vorteilhaft erwiesen, bei intravenöser Applikation Mengen von etwa 0,001 bis 10 mg, vorzugsweise etwa 0,05 bis 5 mg dem Menschen pro Tag zur Erzielung wirksamer Ergebnisse zu verabreichen. Bei oraler Applikation beträgt die Dosierung etwa 0,05 bis 30 mg, vorzugsweise 0,1 bis 10 mg pro Tag beim Menschen.

Die vorstehend genannten Dosierungen werden für die Behandlung der Hypertonie besonders bevorzugt.

Trotzdem kann es gegebenenfalls erforderlich sein, von den genannten Mengen abzuweichen, und zwar in Abhängigkeit vom Körpergewicht beziehungsweise der Art des Applikationsweges, aber auch aufgrund des individuellen Verhaltens gegenüber dem Medikament beziehungsweise der Art von dessen Formulierung und dem Zeitpunkt beziehungsweise Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden kann. Im Falle der Applikation größerer Mengen kann es empfehlenswert sein, diese in mehrere Einzelgaben über den Tag zu verteilen.

Gegenstand der Erfindung sind auch die erfindungsgemäßen Verbindungen zur Behandlung der vorstehenden Krankheiten sowie Verfahren zur Behandlung dieser Krankheiten, in denen diese Verbindungen verwendet werden, sowie ihre Verwendung in Verfahren zur Herstellung von Mitteln, die diese Verbindungen enthalten, zur Behandlung dieser Krankheiten, sowie Verfahren zur Herstellung der Verbindungen.

Gemäß der Erfindung werden pharmazeutische Präparate oder Zusammensetzungen geschaffen, die eine erfindungsgemäße Verbindung oder deren pharmazeutisch verträgliches Salz oder Säureadditionssalz gegebenenfalls zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder parenteral in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Als Hilfsstoffe seien beispielsweise aufgeführt:

Wasser, nicht-toxische organische Lösungsmittel wie Paraffine (z. B. Erdölfraktionen), pflanzliche Öle (z. B. Erdnuß-/Sesam-Öl), Alkohole (z. B. Ethylalkohol, Glycerin), Glykole (z. B. Propylenglykol, Polyethylenglykol), feste Trägerstoffe wie natürliche Gesteinsmehle (z. B. Kaoline, Tonerden, Talkum, Kreide) und synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1 bis 90 Gewichtsprozent enthalten, d. h. in Mengen, die ausreichend sind, um den angegebenen Dosierungsspielraum zu erreichen, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 0,05 bis 10 mg.

Gegenstand der Erfindung ist auch ein Verfahren zur Herstellung von pharmazeutischen Präparaten, dadurch gekennzeichnet, daß man eine oder mehrere Verbindungen gemäß Anspruch 1 oder deren physiologisch verträgliche Salze und gegebenenfalls übliche Trägerstoffe und/oder Verdünnungsmittel vermischt.

Die folgenden Beispiele sollen die Erfindung verdeutlichen:

### Beispiel 1

### 2,2-Dimethyl-6-trifluormethylthio-4-(2'-pyridinyl)--2H-benzo[b]pyran-1'-oxid

Es werden 1,5 g (4,4 mmol) 2,2-Dimethyl-6-trifluormethylthio-4-(2'-pyridinyl)-2H-benzo[b]pyran und 1,4 g (2,4 mmol) 85 %iges Mg-Monoperoxyphthalat-Hexahydrat in einem Gemisch aus 10 ml Ethanol und 5 ml Wasser gelöst und 1 h bei 80° C gerührt. Nach Abkühlen wird mit Wasser verdünnt, zweimal mit Ether extrahiert, die vereinigten organischen Phasen über Magnesiumsulfat getrocknet und eingeengt. Chromatographie an Kieselgel mit Dichlormethan/Ethanol 98:2 ergaben
100 mg (6,4 %) des Pyridin-N-oxids,
Smp. 144-145° C.

### Beispiel 2

### 2,2-Dimethyl-6-trifluormethoxy-4-(2'-pyridinyl)-2H-benzo[b]pyran-l'oxid

Aus 2,0 g (6,2 mmol) 2,2-Dimethyl-6-trifluormethoxy-4-(2'-pyridinyl)-2H-benzo[b]pyran wurden in analoger Weise, wie unter Beispiel 1 beschrieben, 200 mg (9,6 %) des Pyridin-N-oxids, Smp. 168-170° C, erhalten.

### Beispiel 3

2,2-Dimethyl-6-difluormethoxy-4-(2'-pyridinyl)-2H-benzo[b]pyran-1'-oxid

1,9 g (6,3 mmol) 2,2-Dimethyl-6-difluormethoxy-4-(2'-pyridinyl)-2H-benzo[b]pyran und 1,9 g (3,2 mmol) 85 %iges Mg-Monoperoxyphthalat-Hexahydrat werden in 10 ml Eisessig gelöst und 1 h auf 80° C erhitzt. Nach Abkühlen wird mit Natriumhydrogencarbonat-Lösung neutralisiert, mit Dichlormethan extrahiert, die organische Phase über Magnesiumsulfat getrocknet und eingeengt. Nach Chromatographie an Kieselgel mit Dichlormethan/Ethanol 98:2 werden 330 mg (16 %) des Pyridin-N-oxids, Smp. 147-148° C (aus Essigester/Petrolether), erhalten.

### Beispiel 4

2,2-Dimethyl-6-trifluormethylsulfonyl-4-(2'-pyridinyl)-2H-benzo[b]pyran-1'-oxid

Aus 350 mg (0,95 mmol) 2,2-Dimethyl-6-trifluormethylsulfonyl-4-(2'-pyridinyl)-2H-benzo[b]pyran-1'-oxid wurden, wie unter Beispiel 3 beschrieben, 100 mg (27 %) des Pyridin-N-oxids erhalten, farblose Kristalle, Smp. 174-179° C.

### Beispiel 5

2,2-Dimethyl-6-trifluormethoxy-4-(3'-pyridinyl)-2H-benzo[b]pyran-1'-oxid

7,0 g (21,8 mmol) 2,2-Dimethyl-6-trifluormethoxy-4-(3'-pyridinyl)-2H-benzo[b]pyran und 7,7 g (13,2 mmol) 85 %iges Mg-Monoperoxyperphthalat-Hexahydrat werden in einem Gemisch aus 100 ml Wasser und 30 ml Eisessig gelöst und 2 h bei Raumtemperatur verrührt. Es wird mit Ether verdünnt, mit 2 N Natronlauge ausgeschüttelt, die organische Phase über Magnesiumsulfat getrocknet und eingeengt. Nach Chromatographie an Kieselgel mit Dichlormethan/Ethanol 95:5 werden 4,8 g (65 %) des Pyridin-N-oxids als Öl erhalten.

### Beispiel 6

### 2,2-Dimethyl-6-trifluormethylsulfonyl-4-(2'-chinolinyl)-2H-benzo[b]pyran-1'-oxid

Aus 750 mg (1,8 mmol) 2,2-Dimethyl-6-trifluormethylsulfonyl-4-(2'-chinolinyl)-2H-benzo[b]pyran wurden analog Beispiel 3 110 mg (14 %) des Chinolin-N-oxids (farblose Kristalle, Smp. 138-140° C) erhalten.

### Beispiel 7

### 2,2-Dimethyl-6-trifluormethoxy-4-(3'-pyridinyl)-2H-benzo[b]pyran

3,0 g (9,3 mmol) 1-(3'-Pyridinyl)-3-methyl-3-(4'-trifluormethoxyphenoxy)butin werden in 20 ml 1,2-Dichlorbenzol gelöst und 4 h auf 175° C erhitzt. Nach Verdünnen mit Ether wird mit Wasser gewaschen, die organische Phase über Magnesiumsulfat getrocknet und eingeengt. Nach Chromatographie an Kieselgel mit Chloroform als Laufmittel wird das Benzopyran als Öl isoliert, Ausbeute: 2,1 g (70 %).

### Beispiel 8

### 2,2-Dimethyl-6-trifluormethylthio-4-(3'-pyridinyl)-2H-benzo[b]pyran

Aus 800 mg (2,4 mmol) 1-(3'-Pyridinyl)-3-methyl-3-(4'-trifluormethylthiophenoxy)butin werden, wie im vorstehenden Beispiel beschrieben, 250 mg (31 %) des Benzo[b]pyrans als Öl erhalten.

### Beispiel 9

### 2,2-Dimethyl-6-trifluormethylsulfonyl-4-(3'-pyridinyl)-2H-benzo[b]pyran

Analog Beispiel 7 wurden aus 2,7 g (7,3 mmol) 1-(3'-Pyridinyl)-3-methyl-3-(4'-trifluormethylsulfonylphenoxy)butin 1,14 g (42 %) des Benzopyrans erhalten, farblose Kristalle, Smp. 121-122° C (Essigester/Petrolether).

### Beispiel 10

### 2,2-Dimethyl-6-diethoxyphosphoryl-4-(3'-pyridinyl)--2H-benzo[b]pyran

Analog Beispiel 7 werden aus 600 mg (1,61 mmol) 3-Methyl-1-(3'-pyridinyl)-3-(4'-diethoxyphosphorylphenoxy)butin nach Chromatographie an Kieselgel mit Dichlormethan/Ethanol 95:5 360 mg (60 %) des Benzopyrans als Öl erhalten.

### Beispiel 11

### 2,2-Dimethyl-6-trifluormethylsulfonyl-4-(4'-pyridinyl)-2H-benzo[b]pyran

In analoger Weise, wie unter Beispiel 7 beschrieben, wurden aus 2,7 g (7,3 mmol) 1-(4'-Pyridinyl)-3-methyl-3-(4'-trifluormethylsulfonylphenoxy)butin 110 mg (4 %) der Substanz als farblose Kristalle, Smp. 91-93° C, erhalten.

### Beispiel 12

### 2,2-Dimethyl-6-trifluormethoxy-4-(2'-pyridinyl)-3,4-dihydro-2H-benzo[b]pyran-1'-oxid

a) Es werden 700 mg (2,2 mmol) 2,2-Dimethyl-6-trifluormethoxy-4-(2'-pyridinyl)-2H-benzo[b]pyran in einem Gemisch aus 10 ml Ethanol und 1 ml Eisessig gelöst, mit 100 mg 10 % Palladium auf Aktivkohle versetzt und über Nacht in einem Autoklaven bei 50° C und 50 bar hydriert. Nach Abfiltrieren des Katalysators wird eingeengt, der Rückstand in Dichlormethan aufgenommen und mit gesättigter Natriumhydrogencarbonat-Lösung ausgeschüttelt. Die organische Phase wird über Magnesiumsulfat getrocknet und im Vakuum eingeengt. Man erhält 500 mg rohes 2,2-Dimethyl-6-trifluormethoxy-4-(2'-pyridinyl)-3,4-dihydro-2H-benzo[b]pyran, das zur Oxidation weiter eingesetzt wird.
b) Das rohe 3,4-Dihydro-2H-benzo[b]pyran wird in 12 ml Dichlormethan gelöst, mit 350 mg (1,7 mmol) 85 %iger m-Chlorperbenzoesäure versetzt und über Nacht verrührt. Nach Ausschütteln mit gesättigter Natriumhydrogencarbonat-Lösung wird die organische Phase über Magnesiumsulfat getrocknet und eingeengt. Nach Chromatographie an Kieselgel mit Dichlormethan/Ethanol 95:5 werden 275 mg (37 % bezogen auf beide Reaktionsstufen) des Pyridin-N-oxids als Öl erhalten.

### Beispiel 13

### 2,2-Dimethyl-6-trifluormethoxy-4-(2'-cyano-3'-pyridinyl)-2H-benzo[b]pyran

Es werden 1,5 g (4,4 mmol) 2,2-Dimethyl-6-trifluormethoxy-4-(3'-pyridinyl)-2H-benzo[b]pyran-1'-oxid in 10 ml Acetonitril gelöst, 900 mg (8,9 mmol) Triethylamin und 1,34 g (13,5 mmol) Trimethylsilylcyanid hinzugegeben und das Gemisch Über Nacht unter Rückfluß gekocht. Nach Einengen im Vakuum wird der Rückstand in Chloroform aufgenommen, mit Wasser ausgeschüttelt und die organische Phase über Magnesiumsulfat getrocknet. Chromatographie an Kieselgel mit Dichlormethan/Ethanol 98:2 ergeben 320 mg (21 %) der Substanz als farblose Kristalle (Smp. 88-90° C).

### Beispiel 14

2,2-Dimethyl-6-trifluormethoxy-4-(4'-cyano-3'-pyridinyl)-2H-benzo[b]pyran

120 mg (7,8 %) des Nitrils werden aus dem vorstehend beschriebenen Ansatz nach Chromatographie als farblose Kristalle (Smp. 126-127° C, Essigester/Petrolether) erhalten.

### Beispiel 15

### 2,2-Dimethyl-6-trifluormethoxy-4-(4'-chlor-3'-pyridinyl)-2H-benzo[b]pyran

Es werden 1,5 g (4,4 mmol) 2,2-Dimethyl-6-trifluormethoxy-4-(3'-pyridinyl)-2H-benzo[b]pyran-1'-oxid in 7,5 ml Phophoroxychlorid 2 h auf 110° C erhitzt. Nach Abkühlen wird im Vakuum eingeengt, der Rückstand in Chloroform aufgenommen, mit Natriumhydrogencarbonat-Lösung ausgeschüttelt, die organische Phase über Magnesiumsulfat getrocknet und eingeengt. Nach Chromatographie an Kieselgel mit Dichlormethan werden 400 mg (25 %) der Substanz als Öl isoliert.

### Beispiel 16

### 2,2-Dimethyl-6-trifluormethoxy-4-(2'-chlor-3'-pyridinyl)-2H-benzo[b]pyran

Aus dem vorstehend beschriebenen Reaktionsansatz werden 140 mg (9 %) der Substanz nach Chromatographie als Öl erhalten.

### Beispiel 17

### 2,2-Dimethyl-6-trifluormethylsulfonyl-trans-3,4-dihydro-3-hydroxy-4-(2'-pyridinyl)-2H-benzo[b]pyran-1'-oxid

a) 600 mg (1,6 mmol) 2,2-Dimethyl-6-trifluormethylsulfonyl-4-(2'-pyridinyl)-2H-benzo[b]pyran und 1,3 g (6,4 mmol) m-Chlorperbenzoesäure werden in 10 ml Dichlormethan gelöst und 60 h bei Raumtemperatur verrührt. Es wird mit gesättigter Natriumhydrogencarbonat-Lösung ausgeschüttelt, die organische Phase über Magnesiumsulfat getrocknet, eingeengt, an Kieselgel mit Dichlormethan/Ethanol 98:2 chromatographiert und dabei 500 mg (77 %) 2,2-Dimethyl-6-trifluormethylsulfonyl-3,4-epoxy-4-(2'-pyridinyl)-3,4-dihydro-2H-benzo[b]pyran-1'-oxid als Öl isoliert.
b) Unter Argonatmosphäre werden 530 mg (1,7 mmol) Diphenyldiselenid in 6 ml Ethanol gelöst, unter Aufschäumen 130 mg (3,4 mmol) Natriumborhydrid zugegeben und noch 10 min gerührt. Nach Neutralisation mit Eisessig wird zu dieser Lösung eine Lösung von 500 mg (1,25 mmol) des vorstehend beschriebenen Epoxids in 4 ml Ethanol gegeben und 8 h unter Rückfluß gekocht. Nach Abkühlen wird mit Wasser verdünnt, zweimal mit Essigester extrahiert, die organische Phase zweimal mit gesättigter Natriumchlorid-Lösung gewaschen, über Magnesiumsulfat getrocknet und nach Einengen an Kieselgel mit Dichlormethan/Ethanol 98:2 und steigendem Ethanol-Gehalt chromatographiert, wobei 100 mg der im Titel genannten Substanz erhalten werden.

### Beispiel 18

### 2,2-Dimethyl-6-trifluormethoxy-4-(4'-pyrimidinyl)--3,4-dihydro-2H-benzo[b]pyran-3'-oxid

a) Es werden 26,7 g (150 mmol) 4-Trifluormethoxyphenol und 38,0 g (195 mmol) 2-Brom-2-methylpropionsäureethylester in 300 ml Butanon gelöst, mit 30,4 g (0,22 mol) Kaliumcarbonat und 1 g Kaliumiodid versetzt und 26 h unter Rückfluß gekocht. Nach Zugabe von weiteren 13,1 g (67 mmol) des Esters und 10 g (72 mmol) Kaliumcarbonat wird noch 17 h erhitzt, abgekühlt, die anorganischen Salze abfiltriert, das Filtrat eingeengt und der Rückstand mit Dichlormethan an Kieselgel chromatographiert. 31,1 g (71 %) 2-Methyl-2-(4'-trifluormethoxyphenoxy)propionsäureethylester als Öl werden dabei isoliert.
b) 10,1 ml Diisopropylamin werden in 300 ml abs. Tetrahydrofuran gelöst, die Lösung auf -78° C gekühlt, unter Argonatmosphäre 45 ml (72 mmol) einer 1,6 m-Lösung von Butyllithium in Hexan zugetropft und 15 min verrührt. Anschließend wird eine Lösung von 5,64 g (60 mmol) 4-Methylpyrimidin in 120 ml THF zugetropft, auf Raumtemperatur erwärmt, 2 h bei dieser Temperatur gerührt, wieder auf -78° C gekühlt und 17,5 g (60 mmol) des vorstehend beschriebenen Esters in 180 ml THF zugetropft. Es wird über Nacht auf Raumtemperatur erwärmt, mit Wasser versetzt, mit Essigester extrahiert, die organische Phase mit gesättigter Natriumchlorid-Lösung gewaschen, über Magnesiumsulfat getrocknet und eingeengt. Chromatographie des Rückstands an Kieselgel mit Ether ergibt 9,8 g (48 %) 3-Methyl-1-(4'-pyrimidinyl)-3-(4'-trifluormethoxyphenoxy)butanon-2 als öl.
c) Es werden 9,8 g (28,8 mmol) des vorstehend beschriebenen Ketons in 250 ml Ethanol gelöst, mit 1,1 g (29,1 mmol) Natriumborhydrid versetzt und über Nacht bei Raumtemperatur verrührt. Nach Abdestillieren des Lösungsmittels wird mit gesättigter Natriumchlorid-Lösung versetzt, mit Essigester extrahiert, die organische Phase über Magnesiumsulfat getrocknet, eingeengt und der Rückstand an Kieselgel mit Dichlormethan/Ethanol 98:2 chromatographiert. Ausbeute: 8,2 g (83 %) 3-Methyl-1-(4'-pyrimidinyl)-3-(4'-trifluormethoxyphenoxy)butanol-2.
d) 8,2 g (24,0 mmol) des in der vorstehenden Vorschrift beschriebenen Alkohols und 8,0 g (39,4 mmol) 85 %iger m-Chlorperbenzoesäure werden in 150 ml Dichlormethan gelöst und 24 h bei Raumtemperatur verrührt. Der Reaktionsansatz wird gründlich mit gesättigter Natriumhydrogencarbonat-, zweimal mit Natriumchlorid-Lösung gewaschen, die organische Phase über Magnesiumsulfat getrocknet, eingeengt und mit Dichlormethan/Ethanol 96:4 an Kieselgel chromatographiert. Ausbeute: 4,3 g (50 %) 4-(3'-Methyl-3'-(4"-trifluormethoxyphenoxy)-2'-hydroxy)butylpyrimidin-3-oxid.
e) Es werden 4,2 g (11,7 mmol) des vorstehend beschriebenen Alkohols in 20 ml trockenem Triethylamin gelöst, mit 2,6 g (23,3 mmol) Methansulfonylchlorid versetzt und 30 min auf 60° C erwärmt. Nach dem Abkühlen wird mit Wasser versetzt, mit Dichlormethan extrahiert, die organische Phase über Magnesiumsulfat getrocknet, eingeengt und an Kieselgel mit Chloroform chromatographiert. Ausbeute: 300 mg (7,5 %) 3-Methyl-3-(4'-trifluormethoxyphenoxy)-1-(4"-pyrimidinyl)-trans-1-buten-3"-oxid.
f) 300 mg (0,88 mmol) des trans-Butens aus der vorher beschriebenen Reaktionsstufe werden 28 h in 30 ml Toluol unter Rückfluß erhitzt. Nach Abdestillieren des Lösungsmittels in Vakuum wird der Rückstand mit Chloroform an Kieselgel chromatographiert.
   Ausbeute: 100 mg (33 %) 1-(4'-Pyrimidinyl)-1-(2"-hydroxy-5"-trifluormethoxyphenyl)-3-methyl-2-buten -3'-oxid.
g) 100 mg (0,29 mmol) des Phenols aus der vorherigen Reaktionsstufe werden in 10 ml Chloroform gelöst, mit 6 Tropfen konz. Schwefelsäure versetzt und das Gemisch 40 h auf 50° C erwärmt. Nach Abkühlen, Ausschütteln mit Natriumcarbonat-Lösung und Trocknen über Magnesiumsulfat wird mit Chloroform an Kieselgel chromatographiert und 45 mg (45 %) des im Titel genannten Benzo[b]pyrans als Öl erhalten.

### Beispiel 19

### 6-Trifluormethylsulfonyl-2,2-dimethyl-4-methoxy--4-(2'-pyridinyl)-3,4-dihydro-2H-benzo[b]pyran-1'-oxid

a) 1,1 g (7,0 mmol) 2-Brompyridin werden in 20 ml trockenem Tetrahydrofuran unter Argonatmosphäre gelöst, auf -78° C gekühlt und in 20 min mit 4,4 ml (7,0 mmol) einer 1,6 molaren Lösung von Butyllithium in Hexan versetzt. Nach 30 min wird auf -60° C erwärmt, eine Lösung von 2,2 g (8,0 mmol) 6-Trifluormethylthio-2,2-dimethyl-3,4-dihydro-2H-benzo[b]pyran-4-on in 10 ml THF zügig zugespritzt, 2 h bei dieser Temperatur gerührt, auf -78° C gekühlt und über Nacht auf Raumtemperatur erwärmt. Es wird mit gesättigter Ammoniumchlorid-Lösung versetzt, mit Ether extrahiert, die organische Phase über Magnesiumsulfat getrocknet und eingeengt. Nach Chromatographie an Kieselgel mit Dichlormethan werden 330 mg (13 %) 6-Trifluormethylthio-2,2-dimethyl-4-(2'-pyridinyl)-3,4-dihydro-2H-benzo[b]pyran-4-ol erhalten.
b) 300 mg (0,84 mmol) des vorstehend beschriebenen Alkohols werden unter Argonatmosphäre in 10 ml trockenem Tetrahydrofuran gelöst, mit 30 mg (1,0 mmol) 80 %igem Natriumhydrid versetzt und 40 min bei Raumtemperatur verrührt. Nach Zugabe von 240 mg (1,7 mmol) Iodmethan wird noch 2 h gerührt und wie unter a) aufgearbeitet. Ausbeute: 300 mg (96 %) 6-Trifluormethylthio-2,2-dimethyl-4-methoxy-4-(2'-pyridinyl)-3,4-dihydro-2H -benzo[b]pyran.
c) 300 mg (0,81 mmol) des vorstehend beschriebenen Ethers werden mit 200 mg (1,0 mmol) 85 %iger m-Chlorperbenzoesäure in 10 ml Dichlormethan über Nacht verrührt, danach noch einmal die gleiche Menge des Oxidationsmittels zugesetzt und weitere 24 h gerührt. Es wird mit ges. Natriumhydrogencarbonat-Lösung versetzt, mit Dichlormethan ausgeschüttelt, die organische Phase über Magnesiumsulfat getrocknet, eingeengt und der Rückstand an Kieselgel mit Dichlormethan/Ethanol 98:2 chromatographiert. Ausbeute: 130 mg (38 %) des im Titel genannten Sulfons, farblose Kristalle, Smp. 157-158° C.

### Beispiel 20

### 6-Trifluormethylsulfinyl-2,2-dimethyl-4-methoxy--4-(2'-pyridinyl)-3,4-dihydro-2H-benzo[b]pyran-1'-oxid

Bei der im vorstehenden Beispiel beschriebenen Oxidation werden 90 mg (28 %) des Sulfoxids erhalten; farblose Kristalle, Smp. 62-64° C.

### Beispiel 21

### 6-Trifluormethylthio-2,2-dimethyl-4-(2'-methoxy-3'-pyridinyl)-3,4-dihydro-2H-benzo[b]pyran-4-ol

Es werden 1,2 g (5,1 mmol) 3-Iod-2-methoxypyridin unter Argonatmosphäre in 15 ml trockenem Tetrahydrofuran gelöst, auf -90° C gekühlt, 3,1 ml (5,0 mmol) einer 1,6 molaren Lösung von Butyllithium in Hexan zugetropft und 2,5 h bei dieser Temperatur gerührt. Nach Aufwärmen auf -50° C werden 1,5 g (5,4 mmol) 6-Trifluormethylthio-2,2-dimethyl-3,4-dihydro-2H-benzo[b]pyran-4-on in 10 ml THF schnell zugetropft, wieder auf -80° C gekühlt und innerhalb von 2 h auf -40° C erwärmt. Es wird wie unter Umsetzung a) von Beispiel 19 aufgearbeitet und dabei 400 mg (20 %) der Substanz als Öl isoliert.

### Beispiel 22

### 6-Trifluormethylthio-2,2-dimethyl-4-(2'-methoxy-3'--pyridinyl)-2H-benzo[b]pyran

400 mg (1,0 mmol) des vorstehend beschriebenen Alkohols werden mit 490 mg (2,6 mmol) p-Toluolsulfonsäure-Monohydrat in 15 ml Toluol 3 h am Wasserabscheider gekocht. Nach dem Abkühlen wird wie unter Umsetzung c) von Beispiel 19 aufgearbeitet und mit Dichlormethan chromatographiert.
Ausbeute: 270 mg (71 %), farblose Nadeln,
Smp. etwa 25° C.

Die zu den oben aufgeführten Beispielen notwendigen Vorstufen werden folgendermaßen dargestellt:

Die Beispiele 1'-6' wurden in analoger Weise, wie unter Beispiel 7 beschrieben, erhalten und zum Teil ohne intensive Reinigungsoperationen weiter umgesetzt.

### Beispiel 1'

### 2,2-Dimethyl-6-trifluormethylthio-4-(2'-pyridinyl)--2H-benzo[b]pyran

1,8 g (50 %), halbkristalline Substanz aus 3,6 g (10,7 mmol) 1-(2'-Pyridinyl)-3-methyl-3-(4'-trifluormethylthiophenoxy)butin.

### Beispiel 2'

### 2,2-Dimethyl-6-trifluormethoxy-4-(2'-pyridinyl)-2H--benzo[b]pyran

2,0 g (69 %), Kristalle, Smp. 57-59° C, aus 2,9 g (9,0 mmol) 1-(2'-Pyridinyl)-3-methyl-3-(4'-trifluormethoxyphenoxy)butin

### Beispiel 3'

### 2,2-Dimethyl-6-trifluormethylsulfonyl-4-(2'-pyridinyl)-2H-benzo[b]pyran

350 mg (17 %) eines Öls aus 2,0 g (5,4 mmol) 1-(2'-Pyridinyl)-3-methyl-3-(4'-trifluormethylsulfonylphenoxy)butin

### Beispiel 4'

### 2,2-Dimethyl-6-difluormethoxy-4-(2'-pyridinyl)-2H-benzo[b]pyran

1,9 g (46 %) eines Öls aus 4,1 g (13,5 mmol) 1-(2'-Pyridinyl)-3-methyl-3-(4'-difluormethoxyphenoxy)butin

### Beispiel 5'

### 2,2-Dimethyl-6-trifluormethylsulfonyl-4-(2'-chinolinyl)-2H-benzo[b]pyran

Nach Chromatographie mit Dichlormethan an Kieselgel wurden 700 mg (19 %), Kristalle, Smp. 140-142° C (aus Ether/Petrolether), neben 600 mg (16 %) 2-Isopropyl-3-(2'-chinolinyl)-5-trifluormethylsulfonylbenzofuran, Smp. 133-134° C (Ether/Petrolether) aus 3,7 g (8,8 mmol) 1-(2'-Chinolinyl)-3-methyl-3-(4'-trifluormethylsulfonylphenoxy)butin erhalten.

### Beispiel 6'

### 1-(2'-Pyridinyl)-3-methyl-3-(4'-trifluormethylthiophenoxy)butin

Es werden 3,9 g (15 mmol) 3-Methyl-3-(4'-trifluormethylthiophenoxy)butin und 3,1 g (15 mmol) 2-Iodpyridin in 30 ml Triethylamin gelöst, mit 240 mg Bis(triphenylphosphin)palladiumdichlorid und 140 mg Kupfer(I)iodid versetzt und 2,5 h im Schraubdeckelreagenzglas bei 80° C verrührt. Nach Abkühlen wird mit Ether verdünnt, mit Wasser ausgeschüttelt, die organische Phase nochmals mit verd. Salzsäure gewaschen und über Magnesiumsulfat getrocknet. Nach Chromatographie mit Dichlormethan/Ethanol 98:2 an Kieselgel werden 3,6 g (71 %) des Propargylethers als Öl isoliert.

### Beispiel 7'

### 1-(2'-Pyridinyl)-3-methyl-3-(4'-trifluormethoxyphenoxy)butin

7,4 g (30 mmol) 3-Methyl-3-(4'-trifluormethoxyphenoxy)butin, 6,0 g (29,3 mmol) 2-Iodpyridin, 480 mg Bis(triphenylphosphin)palladiumdichlorid und 280 mg Kupfer(I)iodid werden in 50 ml Triethylamin gelöst und 2 h bei Raumtemperatur verrührt. Nach Aufarbeitung, wie vorstehend beschrieben, werden 9,6 g (98 %) des Propargylethers als Öl erhalten.

### Beispiel 8'

### 1-(2'-Pyridinyl)-3-methyl-3-(4'-trifluormethylsulfonylphenoxy)butin

Aus 5,0 g (17,1 mmol) 3-Methyl-3-(4'-trifluormethylsulfonylphenoxy)butin und 3,8 g (18,5 mmol) 2-Iodpyridin wurden analog Beispiel 7' nach 12 h bei Raumtemperatur 2,0 g (32 %) der Substanz als Öl erhalten.

### Beispiel 9'

### 1-(2'-Pyridinyl)-3-methyl-3-(4'-difluormethoxyphenoxy)butin

Aus 3,4 g (15 mmol) 3-Methyl-3-(4'-difluormethoxyphenoxy)butin und 3,1 g (15 mmol) 2-Iodpyridin wurden analog Beispiel 6' 4,1 g (90 %) des Propargylethers als Öl erhalten.

### Beispiel 10'

### 1-(3'-Pyridinyl)-3-methyl-3-(4'-trifluormethylthiophenoxy)butin

500 mg (1,9 mmol) 3-Methyl-3-(4'-trifluormethylthiophenoxy)butin und 390 mg (1,9 mmol) 3-Iodpyridin ergeben analog Beispiel 6' 500 mg (77 %) der Substanz als Öl.

### Beispiel 11'

### 1-(3'-Pyridinyl)-3-methyl-3-(4'-trifluormethoxyphenoxy)butin

12,2 g (50 mmol) 3-Methyl-3-(4'-trifluormethoxyphenoxy)butin, 9,4 g (46 mmol) 3-Iodpyridin, 700 mg Bis(triphenylphosphin)palladiumdichlorid und 500 ml Kupfer(I)iodid werden in 70 ml Triethylamin gelöst und 3 h bei Raumtemperatur verrührt. Es wird, wie unter Beispiel 6' beschrieben, aufgearbeitet und 14,2 g (96 %) der Substanz als Öl isoliert.

### Beispiel 12'

### 1-(3'-Pyridinyl)-3-methyl-3-(4'-trifluormethylsulfonylphenoxy)butin

Aus 2,7 g (9,2 mmol) 3-Methyl-3-(4'-trifluorsulfonylphenoxy)butin, 2,1 g (10,2 mmol) 3-Iod-pyridin, 200 mg Bis(triphenylphosphin)palladiumdichlorid und 100 mg Kupfer(I)iodid in 30 ml Triethylamin werden nach 3 h bei 50° C und Aufarbeitung analog Beispiel 6' 2,7 g (79 %) der Substanz als Öl erhalten.

### Beispiel 13'

### 3-Methyl-1-(3'-pyridinyl)-3-(4'-diethoxyphosphorylphenoxy)butin

Aus 1,0 g (3,37 mmol) 3-Methyl-3-(4'-diethoxyphosphorylphenoxy)butin und 640 mg (3,12 mmol) 3-Iodpyridin werden analog Beispiel 6' nach Chromatographie an Kieselgel mit Ether 620 mg (54 %) der Substanz erhalten.

### Beispiel 14'

### 1-(4'-Pyridinyl)-3-methyl-3-(4'-trifluormethylsulfonylphenoxy)butin

Aus 2,9 g (9,9 mmol) 3-Methyl-3-(4'-trifluormethylsulfonylphenoxy)butin, 2,0 g (9,9 mmol) 4-Iodpyridin, 100 mg Kupfer(I)iodid und 150 mg Bis(triphenylphosphin)palladiumdichlorid in 20 ml Triethylamin werden nach 2 h bei 80° C und bereits beschriebener Aufarbeitung 2,7 g (74 %) des Propargylethers als Öl erhalten.

### Beispiel 15'

### 1-(2'-Chinolinyl)-3-methyl-3-(4'-trifluormethylsulfonylphenoxy)butin

Es werden 3,2 g (10,9 mmol) 3-Methyl-3-(4'-trifluormethylsulfonylphenoxy)butin, 2,6 g (10,2 mmol) 2-Iodchinolin, 140 mg Bis(triphenylphosphin)palladiumdichlorid und 100 mg Kupfer(I)iodid in 20 ml Triethylamin gelöst und das Gemisch 10 min auf 50° C erwärmt. Aufarbeitung analog Beispiel 6' und Chromatographie an Kieselgel mit Dichlormethan ergibt 3,8 g (89 %) der Substanz als Öl.

### Beispiel 16'

### 3-Methyl-3-(4'-trifluormethylthiophenoxy)butin

In einer Lösung von 19,4 g (0,1 mol) 4-(Trifluormethylthio)phenol in 250 ml trockenem Butanon werden 13,8 g (0,1 mol) getrocknetes Kaliumcarbonat und 1,6 g (0,01 mol) Kaliumiodid suspendiert und 15,4 g (0,15 mol) 3-Chlor-3-methyl-1-butin zugetropft. Dann wird unter Rühren 20 Stunden unter Rückfluß erhitzt. Nochmals werden 15,4 g 3-Chlor-3-methyl-1-butin und 13,8 g Kaliumcarbonat zugesetzt, und weiterhin wird 40 Stunden unter Rückfluß erhitzt. Anorganische Bestandteile werden abfiltriert, die Lösung eingeengt, der Rückstand in 200 ml Methylenchlorid aufgenommen und mit 1N NaOH-Lösung extrahiert. Die organische Phase wird mit Wasser gewaschen, getrocknet, eingeengt, und der Rückstand wird über Kieselgel filtriert. Ausbeute: 22 g (85 %)

### Beispiel 17'

### 3-Methyl-3-(4'-trifluormethoxyphenoxy)butin

In einer Lösung von 90 g (0,5 mol) 4-Trifluormethoxyphenol in 900 ml trockenem Aceton werden 69,2 g (0,5 mol) getrocknetes Kaliumcarbonat und 8,3 g (0,05 mol) Kaliumiodid suspendiert, und es werden 70 g (0,68 mol) 3-Chlor-3-methyl-1-butin zugetropft. Nach 36 Stunden Rühren bei Rückflußtemperatur werden nochmals 35 g (0,34 g mol) 3-Chlor-3-methyl-1-butin zugesetzt, und es wird weitere 36 Stunden bei Rückflußtemperatur gerührt. Die abgekühlte Suspension wird filtriert und mit Aceton nachgewaschen. Das Filtrat wird eingeengt, der Rückstand in Methylenchlorid aufgenommen und mit 1N-NaOH-Lösung extrahiert. Die organische Phase wird neutral gewaschen, getrocknet und eingedampft.
Ausbeute: 67 g (55 %)

### Beispiel 18'

### 3-Methyl-3-(4'-trifluormethylsulfonylphenoxy)butin

A) 1 g (4,4 mmol) 4-Trifluormethylsulfonylphenol werden zusammen mit 0,66 g Kaliumcarbonat, 80 mg Kaliumiodid und 2 g 3-Chlor-3-methyl-1-butin in 13 ml trockenem Butanon 20 Stunden unter Argon bei 80-90° C gerührt. Man läßt abkühlen, filtriert und dampft das Filtrat ein. Der Rückstand wird in 20 ml Methylenchlorid aufgenommen, mit Wasser gewaschen (2 x 20 ml), getrocknet und eingedampft. Es bleiben 1,2 g (93 %) des Butins als Öl zurück.
B) 20 g (76,8 mmol) 3-Methyl-3-(4'-trifluormethylthiophenoxy)butin werden in einem Gemisch aus 700 ml Wasser und 700 ml Methanol gelöst und mit 141,8 g Oxone^{R} versetzt. Es wird 4 Tage bei Raumtemperatur verrührt, mit weiteren 500 ml Wasser versetzt und dreimal mit Chloroform extrahiert. Die vereinigten organischen Phasen werden über Magnesiumsulfat getrocknet, eingeengt und der Rückstand mit Dichlormethan an Kieselgel chromatographiert. Ausbeute: 14,0 g (62 %).

### Beispiel 19'

### 3-Methyl-3-(4'-difluormethoxyphenoxy)butin

wird analog Beispiel 16' dargestellt. Ausbeute: 70 %; Sdp. 56-58° C/0,035 mbar.

### Beispiel 20'

### 3-Methyl-3-(4'-diethoxyphosphorylphenoxy)butin

wird analog Beispiel 16' aus 4-Diethoxyphosphorylphenol dargestellt. Ausbeute: 78 %; farbloses Öl nach Chromatographie an Kieselgel mit Essigester/Hexan 40:5.

### Beispiel 21'

### 4-Trifluormethylsulfonylphenol

A) 1 g (5,2 mmol) 4-Trifluormethylthiophenol werden in 20 ml Methanol gelöst, und bei 0° C wird unter Rühren eine Suspension von 9,6 g Oxone^{R} in 20 ml Wasser zugegeben. Nach 5 Tagen Rühren bei Raumtemperatur wird mit 50 ml Wasser verdünnt und mit Chloroform extrahiert (3 x 50 ml). Nach Trocknen und Eindampfen verbleiben 1.1 g (94 %) farbloser Kristalle, Smp. 123° C (Lit.: 119-120° C).
B) 1 g (5,2 mmol) 4-Trifluormethylthiophenol wird zusammen mit 4 ml Eisessig und 4 ml 30 %igem Wasserstoffperoxid 20 Stunden bei 50° C gerührt. Anschließend werden nochmals 2 ml 30 %iges Wasserstoffperoxid zugesetzt, und nach weiteren 2 Stunden bei 50° C wird, wie vorstehend beschrieben, aufgearbeitet. Nach Chromatographie an Kieselgel erhält man 230 mg (20 %) des Phenols.

### Beispiel 22'

### 2,2-Dimethyl-6-trifluormethylthio-3,4-dihydro-2H-benzo[b]pyran-4-on

a) 22,0 g (84,5 mmol) 3-Methyl-3-(4'-trifluormethylthiophenoxy)butin werden in 50 ml 1,2-Dichlorbenzol gelöst, unter Argonatmosphäre 2 h auf 180° C erhitzt und anschließend im Vakuum fraktioniert, wobei 13,0 g (59 %) (Sdp. 55° C/0,02 mbar) 2,2-Dimethyltrifluormethylthio-2H-benzo[b]pyran erhalten werden.
b) 6,5 g (25,0 mmol) 2,2-Dimethyl-6-trifluormethylthio-2H-benzo[b]pyran werden in 25 ml trockenem Chloroform gelöst und auf -5° C gekühlt. Bei dieser Temperatur wird eine Lösung von 4,0 g (1,27 ml; 25,0 mmol) Brom in 20 ml Chloroform zugetropft, noch 5 min gerührt und eingeengt. 2,2-Dimethyl-trans-3,4-dibrom-6-trifluormethylthylthio-3,4-dihydro-2H-benzo[b]pyran kristallisiert in farblosen Kristallen aus kaltem Petrolether. Ausbeute: 10,1 g (96 %), Smp.: 66-67° C.
c) Es werden 1,25 g (54,4 mmol) Natrium in 20 ml absolutem Methanol gelöst, zur Trockne eingeengt, in 1,2-Dimethoxyethan (DME) aufgenommen und mit 22,5 g (53,6 mmol) des vorstehend beschriebenen Dibromids in 60 ml DME versetzt. Anschließend wird 40 min unter Rückfluß gekocht, mit Wasser versetzt, mit Ether ausgeschüttelt und die organische Phase mit gesättigter Natriumchlorid-Lösung gewaschen, über Magnesiumsulfat getrocknet und eingeengt. 2,2-Dimethyl-4-brom-6-trifluormethylthio-2H-benzo[b]pyran fällt als Öl an, das aufgrund seines ¹H-NMR-Spektrums zur weiteren Umsetzung hinreichend rein ist.
   Ausbeute: 16,5 g (91 %).
d) 4,5 g (13,3 mmol) des vorstehend beschriebenen Benzopyrans werden in 10 ml konz. Schwefelsäure gelöst und über Nacht bei Raumtemperatur verrührt. Es wird vorsichtig auf Natriumhydrogencarbonat-Lösung gegeben, mit Ether extrahiert, die organische Phase über Magnesiumsulfat getrocknet und eingeengt. Nach Chromatographie mit Dichlormethan an Kieselgel werden 2,0 g (55 %) des Chromanons erhalten, farblose Kristalle, Smp. 45-47° C.

### Beispiel 23

### Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Hypertonie in Dosierungsmengen von jeweils einer Tablette oder Kapsel einmal täglich geeignet.

| Bestandteile | Gewicht (mg) | |
|---|---|---|
| | Tablette | Kapsel |
| 6-Trifluormethylsulfonyl-2,2-dimethyl-4-(2'-pyridinyl)-2H-benzo[b]pyran-1'-oxid | 0,2 | 0,1 |
| Tragacanth | 10 | |
| Lactose | 247,5 | 300 |
| Maisstärke | 25 | |
| Talk | 15 | |
| Magnesiumstearat | 2,5 | |

### Beispiel 24

### Herstellung von Ampullen

Ampullen, die die im Folgenden genannten Bestandteile enthalten, lassen sich in bekannter Weise herstellen. Der Wirkstoff wird in Wasser und 1,2-Propandiol gelöst und unter Stickstoff in Glasampullen abgefüllt.

| | |
|---|---|
| 6-Trifluormethylsulfonyl-2,2-dimethyl-4-(2'-pyridinyl)-2H-benzo[b]pyran-1'-oxid | 0,02 mg |
| 1,2-Propandiol | 0,8 ml |
| dest. Wasser ad | 2,0 ml |

## Patentansprüche

1. Substituierte Benzopyran-Derivate der allgemeinen Formel I, 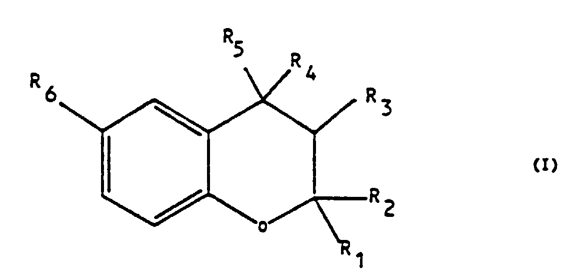 in der
R₁ und R₂, die gleich oder verschieden sein können, Wasserstoff, C₁₋₆-Alkyl, C₃₋₆-verzweigt-Alkyl, C₃₋₇-cycloalkyl, Phenyl oder zusammen mit dem von ihnen eingeschlossenen Kohlenstoffatom C₃₋₇-Spiroalkyl bedeuten,
entweder R₃ oder R₄ Wasserstoff, Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und der jeweils andere Substituent der beiden Wasserstoff ist, oder R₃ und R₄ zusammen eine Bindung bilden,
R₅ eine monocyclische sechsgliedrige oder eine bicyclische aus zwei kondensierten Sechs ringen bestehende Aryl- oder N-Heteroarylgruppe, in der der Heterocyclus ein oder zwei Stickstoffatome enthält, bedeutet, die einer der folgenden zwei Untergruppen A) oder B) angehört, wobei in
A) R₅ eine N-Heteroarylgruppe mit einem oder zwei N-Atomen ist, die in der 2-Stellung eine N-Oxidgruppe trägt und gegebenenfalls ein- oder zweifach durch Halogen, C₁₋₈-Alkyl, C₁₋₈-Alkoxy, C₁₋₈-Mono- oder C₁₋₈-Dialkylamino, Hydroxy, Amino, Cyan, C₁₋₈-Alkoxycarbonyl, C₁₋₈-Mono- oder C₁₋₈-Dialkylaminocarbonyl, Hydroxycarbonyl, Aminocarbonyl, Phenyl substituiert ist, und in
B) R₅ eine N-Heteroarylgruppe oder ein N-Heteroaryl-N-oxid ist, deren Grundkörper aus der Gruppe 3-Pyridinyl, 4-Pyridinyl, 5-Pyrimidinyl, 3-Pyridazinyl, 4-Pyrimidinyl, 2-Pyrazinyl, 3-Chinolinyl, 4-Isochinolinyl stammt und gegebenenfalls ein- oder zweifach durch Halogen, C₁₋₈-Alkyl, C₁₋₈-Alkoxy, C₁₋₈-Monoalkylamino, C₁₋₈-Dialkylamino, Cyan, Hydroxy, Amino, Phenyl substituiert ist, wobei die Hydroxy- oder die N-Oxidgruppe in dieser Untergruppe sich nicht in der 2-Stellung befindet, und
R₆ Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl, Trifluorethylsulfonyl, Phosphono oder C₁₋₈-Dialkoxyphosphoryl ist, wobei vorstehend genanntes Alkyl geradkettig oder verzweigt sein kann, sowie deren Salze und Säureadditionssalze, Tautomere und optische Isomere.

2. Verbindungen gemäß Anspruch 1, worin R₅ die Gruppe A) bedeutet.

3. Verbindungen gemäß Anspruch 1, worin R₅ die Gruppe B) bedeutet.

4. Verbindungen gemäß Anspruch 2 oder 3, worin R₃ und R₄ zusammen eine Bindung bilden.

5. 6-Difluormethoxy-2,2-dimethyl-4-(2'-pyridinyl)-2H-benzo(b)pyran-1'-oxid gemäß Anspruch 1.

6. 6-Trifluormethoxy-2,2-dimethyl-4-(2'-pyridinyl)-2H-benzo(b)pyran-1'-oxid gemäß Anspruch 1.

7. 6-Trifluormethylthio-2,2-dimethyl-4-(2'-pyridinyl)-2H-benzo(b)pyran-1'-oxid gemäß Anspruch 1.

8. 6-Trifluormethylsulfonyl-2,2-dimethyl-4-(2'-pyridinyl)-2H-benzo(b)pyran-1'-oxid gemäß Anspruch 1.

9. 6-Trifluormethylthio-2,2-dimethyl-4-(3'-pyridinyl)-2H-benzo(b)pyran gemäß Anspruch 1.

10. 6-Trifluormethylsulfonyl-2,2-dimethyl-4-(3'-pyridinyl)-2H-benzo(b)pyran gemäß Anspruch 1.

11. Verfahren zur Herstellung der Verbindungen der Formel I gemäß Anspruch 1 und ihrer Salze sowie Säureadditionssalze dadurch gekennzeichnet, daß man
a) zur Herstellung einer Verbindung der Formel I, in der R₅ eine N-Heteroarylgruppe daurstellt, die eine N-Oxidgruppe in der 2-, 3- oder 4-Position trägt, eine Verbindung der allgemeinen Formel I, in der R₁, R₂, R₃, R₄ und R₆ die vorstehend angegebene Bedeutung besitzen und R₅ eine entsprechende N-Heteroarylgruppe mit einem N-Atom in 2-, 3- bzw. 4-Stellung ist, oxidiert, oder
b) zur Herstellung einer der Formel VI entsprechenden Verbindung der Formel I, 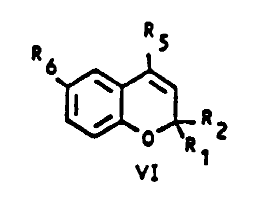 in der R₁, R₂, R₅ und R₆ die oben angegebene Bedeutung haben und R₃ und R₄ zusammen eine Bindung bilden, eine Verbindung der allgemeinen Formel II 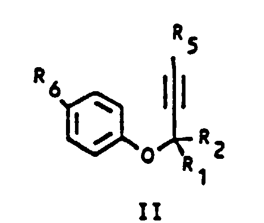 mit der oben angegebenen Bedeutung von R₁, R₂, R₅ und R₆, erhitzt oder aus einer der Formel III entsprechenden Verbindung der Formel I, worin R₃ Wasserstoff und R₄ Hydroxy bedeutet, 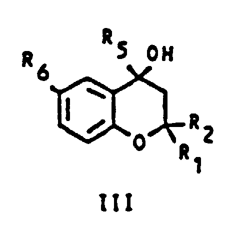 mit der angegebenen Bedeutung von R₁, R₂, R₅ und R₆ Wasser abspaltet, oder
c) zur Herstellung einer der allgemeinen Formel III entsprechenden Verbindung der Formel I mit der oben angegebenen Bedeutung von R₁, R₂, R₅ und R₆, eine Verbindung der allgemeinen Formel IV, 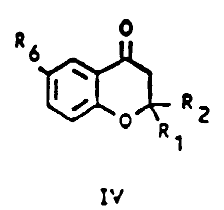 in der R₁, R₂ und R₆ die oben angegebene Bedeutung haben, mit einer metallorganischen Verbindung R₅M umsetzt, in der R₅ die vorstehend angegebene Bedeutung hat und M ein Metall oder Metallhalogenid, vorzugsweise Li-oder MgHal darstellt, wobei Hal Chlor, Brom oder Jod ist, oder
d) zur Herstellung solcher Verbindungen der allgemeinen Formel I, in der R₃ und R₄ Wasserstoff bedeuten, in der R₁, R₂, R₅ und R₆ die oben angegebene Bedeutung haben, eine Verbindung der allgemeinen Formel V, 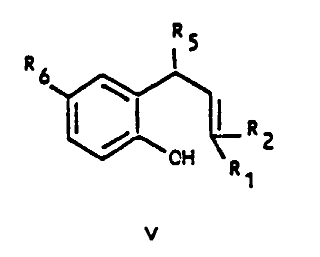 in der R₁, R₂, R₅ und R₆ die oben angegebene Bedeutung haben, cyclisiert, oder eine Verbindung der allgemeinen Formel VI, 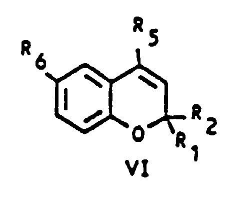 in der R₁, R₂, R₅ und R₆ die angegebene Bedeutung haben, in Gegenwart eines Katalysators hydriert, oder
e) zur Herstellung einer Verbindung der allgemeinen Formel I, in der R₆ Difluormethylsulfinyl, Difluormethylsulfonyl, Trifluormethylsulfinyl, Trifluormethylsulfonyl, Trifluorethylsulfinyl oder Trifluorethylsulfonyl bedeutet und R₁, R₂, R₃, R₄ und R₅ die vorstehend angegebene Bedeutung haben, eine Verbindung der allgemeinen Formel I, in der R₆ Difluormethylthio, Trifluormethylthio bzw. Trifluorethylthio ist, oxidiert, oder
f) zur Herstellung einer Verbindung der allgemeinen Formel I, in der R₅ einen N-Heteroaromaten, der in o- oder p-Stellung zum N-Atom durch Chlor oder Cyano substituiert ist, und R₃ und R₄ jeweils Wasserstoff oder zusammen eine Bindung bedeuten und R₁, R₂ und R₆ die oben angegebene Bedeutung haben, eine Verbindung der allgemeinen Formel I, in der R₅ eine N-Heteroaryl-N-Oxidgruppe ist und R₁, R₂, R₃, R₄ und R₆ die entsprechende Bedeutung haben, mit einem Nichtmetallchlorid bzw. einem silylcyanid umsetzt, oder
g) zur Herstellung einer Verbindung der Formel I, in der R₃ Hydroxy und R₄ Wasserstoff ist, und R₁, R₂, R₅ und R₆ die oben angegebene Bedeutung haben, eine Verbindung der Formel I, in der R₁, R₂, R₅ und R₆ die oben angegebene Bedeutung haben, und R₃ und R₄ zusammen eine Bindung bilden, zu dem Epoxid epoxidiert und anschließend das Epoxid zu der Verbindung der Formel I reduziert, oder
h) zur Herstellung von Verbindungen der Formel I, in der R₁, R₂, R₅ und R₆ die oben angegebene Bedeutung haben und R₃ oder R₄ C₁₋₈-Alkoxy, Formyloxy, C₁₋₈-Alkylcarbonyloxy, C₁₋₈-Alkoxycarbonyloxy, C₁₋₈-Mono-alkylamonocarbonyloxy oder C₁₋₈-Dialkylamino-carbonyloxy bedeuten, die entsprechenden Verbindungen der Formel I, in der R₃ oder R₄ Hydroxy bedeuten, mit entsprechenden Alkylierungsmitteln oder Acylierungsmitteln umsetzt, und gegebenenfalls nach den vorstehenden Verfahren erhaltene Verbindungen in ihre Salze oder Säureadditionssalze oder N-Oxide überführt.

12. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

13. Verbindungen der Formeln II, IV, V und XI mit der in Anspruch 1 angegebenen Bedeutung der Substituenten:

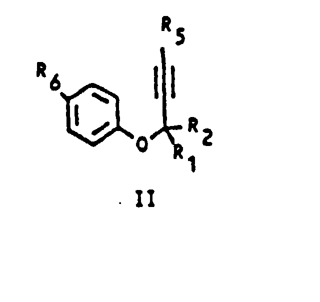

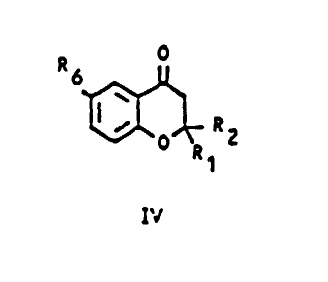

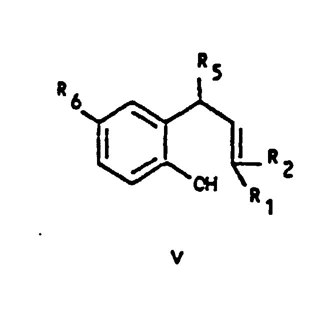

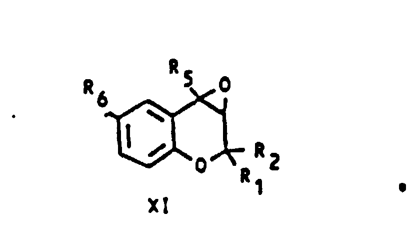

14. Verwendung von einer oder mehreren Verbindungen gemäß Anspruch 1 oder deren physiologisch verträglichen Salzen zur Herstellung eines Mittels zur Prophylaxe und Behandlung von Bluthochdruck, Herzinsuffizienz, Angina pectoris, peripheren Durchblutungsstörungen, cerebraler Insuffizienz, Asthma und akuten und chronischen Herzkrankheiten.

## Claims

1. Substituted benzopyran derivatives of the general formula I 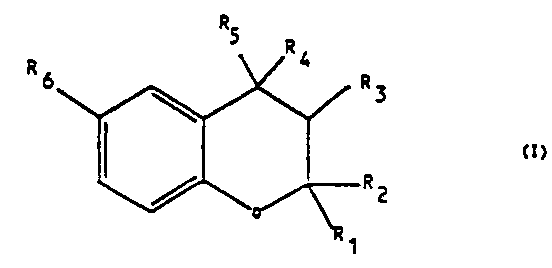 in which
R₁ and R₂, which may be identical or different, denote hydrogen, C₁₋₆-alkyl, branched C₃₋₆-alkyl, C₃₋₇-cycloalkyl or phenyl or denote, together with the carbon atom enclosed by them, C₃₋₇-spiroalkyl,
either R₃ or R₄ denotes hydrogen, hydroxyl, C₁₋₈-alkoxy, formyloxy, C₁₋₈-alkylcarbonyloxy, C₁₋₈-alkoxycarbonyloxy, C₁₋₈-monoalkylaminocarbonyloxy or C₁₋₈-dialkylaminocarbonyloxy, where the C₁₋₈-alkyl or -alkoxy groups may be either linear or branched, and the other substituent of the two in each case is hydrogen, or R₃ and R₄ together form a bond,
R₅ denotes a monocyclic six-membered aryl or N-heteroaryl group, a bicyclic aryl or N-heteroaryl group which is composed of two fused six-membered rings, in which the heterocycle contains one or two nitrogen atoms which belongs to one of the following two subgroups A) or B), in which context in
A) R₅ is an N-heteroaryl group containing one or two N atoms, which carries an N-oxide group in the 2 position and is optionally substituted one or two times by halogen, C₁₋₈-alkyl, C₁₋₈-alkoxy, C₁₋₈-mono- or C₁₋₈-dialkylamino, hydroxyl, amino, cyano, C₁₋₈-alkoxycarbonyl, C₁₋₈-mono- or C₁₋₈-dialkylaminocarbonyl, hydroxycarbonyl, aminocarbonyl or phenyl, and in
B) R₅ is an N-heteroaryl group or an N-heteroaryl N-oxide whose basic structure derives from the group 3-pyridyl, 4-pyridyl, 5-pyrimidinyl, 3-pyridazinyl, 4-pyrimidinyl, 2-pyrazinyl, 3-quinolyl and 4-isoquinolyl and is optionally substituted one or two times by halogen, C₁₋₈-alkyl, C₁₋₈-alkoxy, C₁₋₈-monoalkylamino, C₁₋₈-dialkylamino, cyano, hydroxyl, amino or phenyl, the hydroxyl group or the N-oxide group in this subgroup not being located in the 2 position, and
R₆ is difluoromethoxy, trifluoromethoxy, trifluoroethoxy, tetrafluoroethoxy, difluoromethylthio, difluoromethylsulphinyl, difluoromethylsulphonyl, trifluoromethylthio, trifluoromethylsulphinyl, trifluoromethylsulphonyl, trifluoroethylthio, trifluoroethylsulphinyl, trifluoroethylsulphonyl, phosphono or C₁₋₈-dialkoxyphosphoryl, in which abovementioned alkyl can be straight-chain or branched, and their salts and acid addition salts, tautomers and optical isomers.

2. Compounds according to Claim 1, in which R₅ denotes the group A).

3. Compounds according to Claim 1, in which R₅ denotes the group B).

4. Compounds according to Claim 2 or 3, in which R₃ and R₄ together form a bond.

5. 6-Difluoromethoxy-2,2-dimethyl-4-(2'-pyridyl)-2H-benzo[b]pyran 1'-oxide according to Claim 1.

6. 6-Trifluoromethoxy-2,2-dimethyl-4-(2'-pyridyl)-2H-benzo[b]pyran 1'-oxide according to Claim 1.

7. 6-Trifluoromethylthio-2,2-dimethyl-4-(2'-pyridyl)-2H-benzo[b]pyran 1'-oxide according to Claim 1.

8. 6-Trifluoromethylsulphonyl-2,2-dimethyl-4-(2'-pyridyl)-2H-benzo[b]pyran 1'-oxide according to Claim 1.

9. 6-Trifluoromethylthio-2,2-dimethyl-4-(3'-pyridyl)-2H-benzo[b]pyran according to Claim 1.

10. 6-Trifluoromethylsulphonyl-2,2-dimethyl-4-(3'-pyridyl)-2H-benzo[b]pyran according to Claim 1.

11. Process for the preparation of the compounds of the formula I according to Claim 1 and their salts and acid addition salts, characterized in that
a) for the preparation of a compound of the formula I in which R₅ represents an N-heteroaryl group which carries an N-oxide group in the 2, 3 or 4 position, a compound of the general formula I in which R₁, R₂, R₃, R₄ and R₆ have the abovementioned meaning and R₅ is a corresponding N-heteroaryl group having an N atom in 2, 3 or 4 position is oxidized, or
b) for the preparation of a compound of the formula I which corresponds to the formula VI 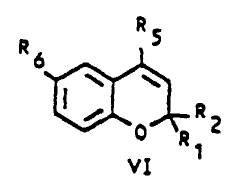 in which R₁, R₂, R₅ and R₆ have the meaning given above and R₃ and R₄ together form a bond, a compound of the general formula II 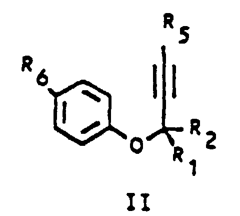 having the meaning given above of R₁, R₂, R₅ and R₆ is heated, or water is eliminated from a compound of the formula I which corresponds to the formula III and in which R₃ denotes hydrogen and R₄ denotes hydroxyl 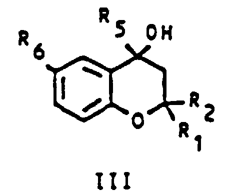 having the given meaning of R₁, R₂, R₅ and R₆, or
c) for the preparation of a compound of the formula I which corresponds to the general formula III and has the meaning given above of R₁, R₂, R₅ and R₆, a compound of the general formula IV 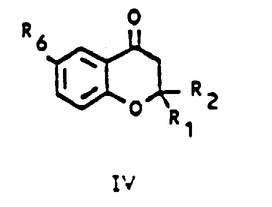 in which R₁, R₂ and R₆ have the meaning given above is reacted with an organometallic compound R₅M in which R₅ has the abovementioned meaning and M represents a metal or metal halide, preferably Li- or MgHal, where Hal is chlorine, bromine or iodine, or
d) for the preparation of those compounds of the general formula I in which R₃ and R₄ denote hydrogen, in which R₁, R₂, R₅ and R₆ have the meaning given above, a compound of the general formula V 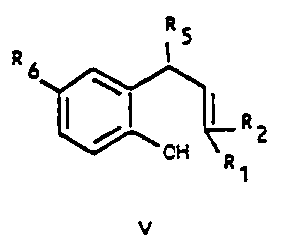 in which R₁, R₂, R₅ and R₆ have the meaning given above is cyclized, or a compound of the general formula VI 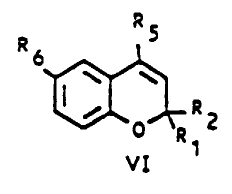 in which R₁, R₂, R₅ and R₆ have the given meaning is hydrogenated in the presence of a catalyst, or
e) for the preparation of a compound of the general formula I in which R₆ denotes difluoromethylsulphinyl, difluoromethylsulphonyl, trifluoromethylsulphinyl, trifluoromethylsulphonyl, trifluoroethylsulphinyl or trifluoroethylsulphonyl and R₁, R₂, R₃, R₄ and R₅ have the abovementioned meaning, a compound of the general formula I in which R₆ is difluoromethylthio, trifluoromethylthio or trifluoroethylthio is oxidized, or
f) for the preparation of a compound of the general formula I in which R₅ denotes an N-heteroaromatic radical which is substituted in the o or p position relative to the N atom by chlorine or cyano, and R₃ and R₄ in each case denote hydrogen or together denote a bond and R₁, R₂ and R₆ have the meaning given above, a compound of the general formula I in which R₅ is an N-heteroaryl N-oxide group and R₁, R₂, R₃, R₄ and R₆ have the appropriate meaning is reacted with a nonmetal chloride or a silyl cyanide, or
g) for the preparation of a compound of the formula I in which R₃ is hydroxyl and R₄ is hydrogen, and R₁, R₂, R₅ and R₆ have the meaning given above, a compound of the formula I in which R₁, R₂, R₅ and R₆ have the meaning given above, and R₃ and R₄ together form a bond, is epoxidized to give the epoxide and then the epoxide is reduced to the compound of the formula I, or
h) for the preparation of compounds of the formula I in which R₁, R₂, R₅ and R₆ have the meaning given above and R₃ or R₄ denote C₁₋₈-alkoxy, formyloxy, C₁₋₈-alkylcarbonyloxy, C₁₋₈-alkoxycarbonyloxy, C₁₋₈-monoalkylaminocarbonyloxy or C₁₋₈-dialkylaminocarbonyloxy, the corresponding compounds of the formula I in which R₃ or R₄ denotes hydroxyl are reacted with corresponding alkylating agents or acylating agents, and compounds obtained by the above processes are optionally converted into their salts or acid addition salts or N-oxides.

12. Pharmaceutical preparation which contains one or more of the compounds according to Claim 1 or their physiologically tolerated salts and, if appropriate, conventional excipients and/or diluents.

13. Compounds of the formulae II, IV, V and XI having the meaning as given in Claim 1 for the substituents:

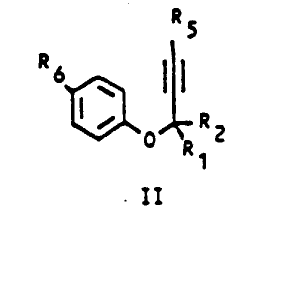

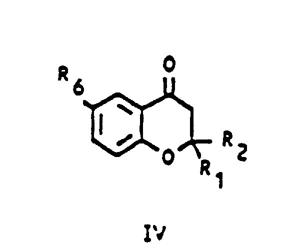

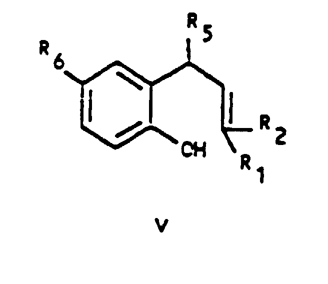

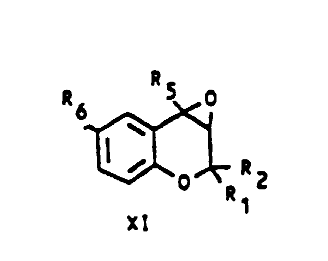

14. Use of one or more compounds according to Claim 1 or their physiologically tolerated salts for the preparation of an agent for the prophylaxis and treatment of high blood pressure, cardiac insufficiency, angina pectoris, peripheral circulatory disorders, cerebral insufficiency, asthma, and acute and chronic heart disease.

## Revendications

1. Dérivés substitués du benzopyranne répondant à la Formule générale I, 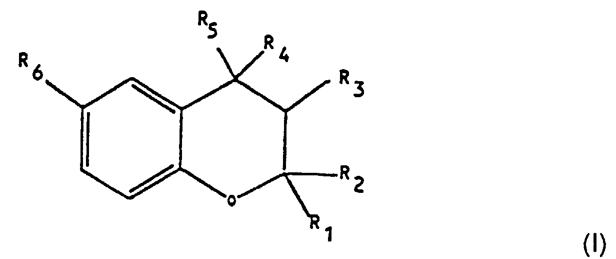 dans laquelle
R₁ et R₂, qui peuvent être identiques ou différents, représentent un atome d'hydrogène, un groupe alkyle en C₁₋₆, un groupe alkyle ramifié en C₃₋₆, un groupe cycloalkyle en C₃₋₇, un groupe phényle ou, ensemble avec l'atome de carbone qu'ils entourent, ils signifient un groupe spiroalkyle en C₃₋₇,
soit R₃, soit R₄ représente un atome d'hydrogène, un groupe hydroxyle, un groupe alcoxy en C₁₋₈, un groupe formyloxy, un groupe (alkyle en C₁₋₈)carbonyloxy un groupe (alcoxy en C₁₋₈)carbonyloxy un groupe mono(alkyle en C₁₋₈)aminocarbonyloxy ou un groupe di(alkyle en C₁₋₈)aminocarbonyloxy, où les groupes alkyles ou alcoxy en C₁₋₈ peuvent être tant linéaires que ramifiés, et l'autre substituant parmi les deux représente un atome d'hydrogène ou R₃ et R₄ forment ensemble une liaison,
R₅ représente un groupe aryle ou N-hétéroaryle monocyclique à six membres ou bicyclique constitué de deux noyaux à six membres condensés, dans lequel l'hétérocycle contient un ou deux atomes d'azote, qui appartient à un des deux sous-groupes suivants A) ou B), où dans
A) R₅ représente un groupe N-hétéroaryle avec un ou deux atomes d'azote, qui portent en position 2 un groupe N-oxyde et qui est substitué éventuellement une ou deux fois par un atome d'halogène, un groupe alkyle en C₁₋₈, un groupe alcoxy en C₁₋₈, un groupe monoalkyle en C₁₋₈- ou di(alkyle en C₁₋₈)amino, un groupe hydroxyle, un groupe amino, un groupe cyano, un groupe (alcoxy en C₁₋₈)carbonyle, un groupe mono(alkyle en C₁₋₈)- ou di(alkyle en C₁₋₈)aminocarbonyle, un groupe hydroxycarbonyle, un groupe aminocarbonyle, un groupe phényle, et dans
B) R₅ représente un groupe N-hétéroaryle ou un groupe N-hétéroaryl-N-oxyde, dont le squelette provient du groupe constitué du groupe 3-pyridinyle, du groupe 4-pyridinyle, du groupe 5-pyrimidinyle, du groupe 3-pyridasinyle, du groupe 4-pyrimidinyle, du groupe 2-pyrasinyle, du groupe 3-quinolinyle, du groupe 4-isoquinolinyle et qui est substitué éventuellement une ou deux fois par un atome d'halogène, un groupe alkyle en C₁₋₈, un groupe alcoxy en C₁₋₈, un groupe mono(alkyle en C₁₋₈)amino, un groupe di(alkyle en C₁₋₈)amino, un groupe cyano, un groupe hydroxyle, un groupe amino, un groupe phényle; le groupe hydroxyle ou le groupe N-oxyde dans ce sous-groupe ne se trouvant pas en position 2, et
R₆ représente un groupe difluorométhoxy, un groupe trifluorométhoxy, un groupe trifluoroéthoxy, un groupe tétrafluoroéthoxy, un groupe difluorométhylthio, un groupe difluorométhylsulfinyle, un groupe difluorométhylsulfonyle, un groupe trifluorométhylthio, un groupe trifluorométhylsulfinyle, un groupe trifluorométhylsulfonyle, un groupe trifluoroéthylthio, un groupe trifluoroéthylsulfinyle, un groupe trifluoroéthylsulfonyle, un groupe phosphono ou un groupe di(alcoxy en C₁₋₈)phosphoryle, le groupe alkyle précité pouvant être linéaire ou ramifié, de même que leurs sels et leurs sels d'addition acide, leurs tautomères et leurs isomères optiques.

2. Composés selon la revendication 1, dans lesquels R₅ représente le groupe A).

3. Composés selon la revendication 1, dans lesquels R₅ représente le groupe B).

4. Composés selon la revendication 2 ou 3, dans lesquels R₃ et R₄ forment ensemble une liaison.

5. 6-Difluorométhoxy-2,2-diméthyl-4-(2'-pyridinyl)-2H-benzo(b)pyranne-1'-oxyde selon la revendication 1.

6. 6-Trifluorométhoxy-2,2-diméthyl-4-(2'-pyridinyl)-2H-benzo(b)pyranne-1'-oxyde selon la revendication 1.

7. 6-Trifluorométhylthio-2,2-diméthyl-4-(2'-pyridinyl)-2H-benzo(b)-pyranne-1'-oxyde selon la revendication 1.

8. 6-Trifluorométhylsulfonyl-2,2-diméthyl-4-(2'-pyridinyl)-2H-benzo(b)-pyranne-1'-oxyde selon la revendication 1.

9. 6-Trifluorométhylthio-2,2-diméthyl-4-(3'-pyridinyl)-2H-benzo(b)-pyranne selon la revendication 1.

10. 6-Trifluorométhylsulfonyl-2,2-diméthyl-4-(3'-pyridinyl)-2H-benzo(b)-pyranne selon la revendication 1.

11. Procédé de préparation des composés répondant à la Formule I selon la revendication 1, et de leurs sels, de même que de leurs sels d'addition acide, caractérisé en ce que
a) on oxyde, pour la préparation d'un composé répondant à la Formule I, dans laquelle R₅ représente un groupe N-hétéroaryle, qui porte un groupe N-oxyde en position 2, 3 ou 4, un composé répondant à la Formule générale I, dans laquelle R₁, R₂, R₃, R₄ et R₆ présentent la signification indiquée ci-dessus et R₅ représente un groupe N-hétéroaryle correspondant avec un atome d'azote en position 2, 3, respectivement 4, ou
b) on chauffe, pour la préparation d'un composé répondant à la Formule I correspondant à la Formule VI, 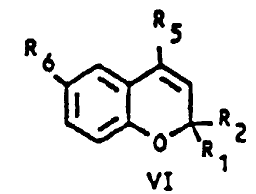 dans laquelle R₁, R₂, R₅ et R₆ présentent la signification indiquée ci-dessus et R₃ et R₄ forment ensemble une liaison, un composé répondant à la Formule générale II 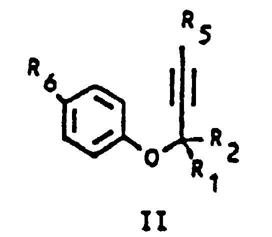 avec la signification indiquée ci-dessus pour R₁, R₂, R₅ et R₆, ou on libère de l'eau à partir d'un composé répondant à la Formule I correspondant à la Formule III, dans laquelle R₃ représente un atome d'hydrogène et R₄ représente un groupe hydroxyle, 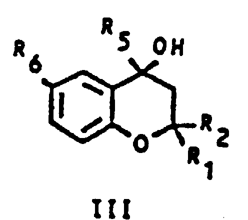 avec la signification indiquée pour R₁, R₂, R₅ et R₆, ou
c) on fait réagir, pour la préparation d'un composé répondant à la Formule I correspondant à la Formule générale III avec la signification indiquée ci-dessus pour R₁, R₂, R₅ et R₆, un composé répondant à la Formule générale IV, 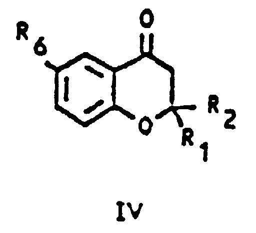 dans laquelle R₁, R₂ et R₆ présentent la signification indiquée ci-dessus, avec un composé organométallique R₅M, dans lequel R₅ présente la signification indiquée précédemment et M représente un métal ou un halogénure métallique, de préférence Li- ou MgHal, où Hal représente un atome de chlore, un atome de brome ou un atome d'iode, ou
d) on cyclise, pour la préparation de composés répondant à la Formule générale I, dans laquelle R₃ et R₄ représentent un atome d'hydrogène, dans laquelle R₁, R₂, R₅ et R₆ présentent la signification indiquée ci-dessus, un composé répondant à la Formule générale V, 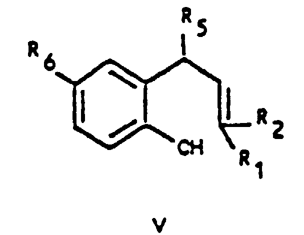 dans laquelle R₁, R₂, R₅ et R₆ présentent la signification indiquée ci-dessus, ou on hydrogène un composé répondant à la Formule générale VI, 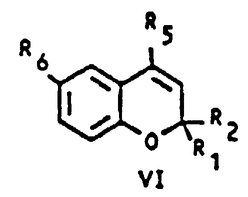 dans laquelle R₁, R₂, R₅ et R₆ présentent la signification indiquée, en présence d'un catalyseur, ou
e) on oxyde, pour la préparation d'un composé répondant à la Formule générale I, dans laquelle R₆ représente un groupe difluorométhylsulfinyle, un groupe difluorométhylsulfonyle, un groupe trifluorométhylsulfinyle, un groupe trifluorométhylsulfonyle, un groupe trifluoroéthylsulfinyle ou un groupe trifluoroéthylsulfonyle et R₁, R₂, R₃, R₄ et R₅ présentent la signification indiquée ci-dessus, un composé répondant à la Formule générale I, dans laquelle R₆ représente un groupe difluorométhylthio, un groupe trifluorométhylthio, respectivement un groupe trifluoroéthylthio, ou
f) on fait réagir, pour la préparation d'un composé répondant à la Formule générale I, dans laquelle R₅ représente une fraction N-hétéroaromatique, qui est substituée en position o ou p par rapport à l'atome d'azote par un atome de chlore ou par un groupe cyano, et R₃ et R₄ représentent respectivement un atome d'hydrogène ou forment ensemble une liaison et R₁, R₂ et R₆ présentent la signification indiquée ci-dessus, un composé répondant à la Formule générale I, dans laquelle R₅ représente un groupe N-hétéroaryl-N-oxyde et R₁, R₂, R₃, R₄ et R₆ présentent la signification correspondante, avec un chlorure non-métallique respectivement un cyanure silylique, ou
g) on époxyde, pour la préparation d'un composé répondant à la Formule I, dans laquelle R₃ représente un groupe hydroxyle et R₄ représente un atome d'hydrogène, et R₁, R₂, R₅ et R₆ présentent la signification indiquée ci-dessus, un composé répondant à la Formule générale I, dans laquelle R₁, R₂, R₅ et R₆ présentent la signification indiquée ci-dessus et R₃ et R₄ forment ensemble une liaison, en époxyde et on réduit ensuite l'époxyde en composé répondant à la Formule I, ou
h) on fait réagir, pour la préparation de composés répondant à la Formule I, dans laquelle R₁, R₂, R₅ et R₆ présentent la signification indiquée ci-dessus et R₃ ou R₄ représente un groupe alcoxy en C₁₋₈, un groupe formyloxy, un groupe (alkyle en C₁₋₈)carbonyloxy, un groupe (alcoxy en C₁₋₈)carbonyloxy, un groupe mono(alkyle en C₁₋₈)aminocarbonyloxy ou un groupe di(alkyle en C₁₋₈)aminocarbonyloxy, les composés correspondants répondant à la Formule I, dans laquelle R₃ ou R₄ représente un groupe hydroxyle, avec des agents d'alkylation ou des agents d'acylation correspondants, et on convertit éventuellement les composés obtenus suivant les procédés précédents en leurs sels ou sels d'addition acide ou N-oxydes.

12. Préparation pharmaceutique, caractérisée en ce qu'elle contient un ou plusieurs composés selon la revendication 1, ou leurs sels physiologiquement acceptables et éventuellement des supports et/ou des diluants courants.

13. Composés répondant aux Formules II, IV, V et XI, avec la signification des substituants indiquée à la revendication 1 :

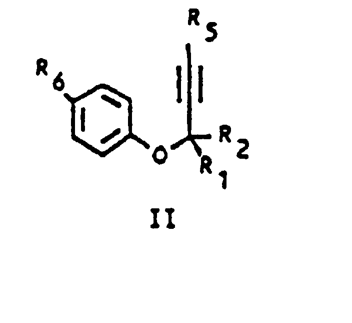

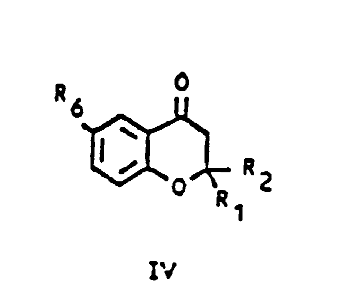

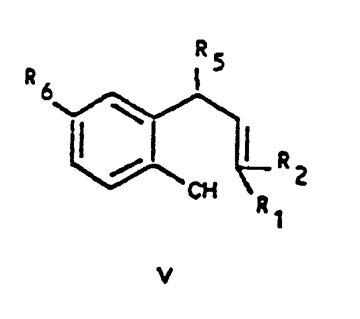

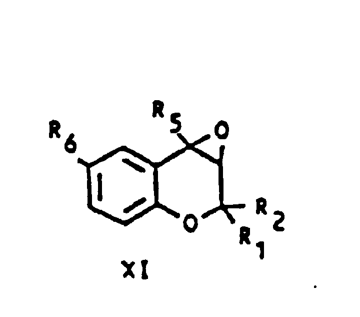

14. Utilisation d'un ou de plusieurs composés selon la revendication 1 ou de leurs sels physiologiquement acceptables pour la préparation d'un médicament pour la prophylaxie et le traitement de l'hypertension, de l'insuffisance cardiaque, de l'angine de poitrine, des troubles de la circulation sanguine périphérique, de l'insuffisance cérébrale, de l'asthme et des maladies cardiaques aiguës et chroniques.
